# EUROPEAN PATENT SPECIFICATION

(11) **EP 0 662 948 B1**
(45) Date of publication and mention of the grant of the patent: **07.10.1998**
(21) Application number: 94921642.8
(22) Date of filing: 12.07.1994
(51) Int. Cl.: C07C 229/36, A61K 31/195

(54) **2-AMINO-4-PHENYL-4-OXO-BUTYRIC ACID DERIVATIVES WITH KYNURENINASE AND/OR KYNURENINE-3-HYDROXYLASE INHIBITING ACTIVITY**
2-AMINO-4-PHENYL-4-OXO-BUTTERSÄURE-DERIVATE MIT KYNURENINASE UND/ODER KYNURENIN-3-HYDROXYLASE INHIBIERENDER WIRKUNG
DERIVES DE L'ACIDE 2-AMINO-4-PHENYL-4-OXOBUTYRIQUE, AYANT UNE ACTIVITE INHIBITRICE DE LA CYNURENINASE ET/OU DE LA CYNURENINE-3-HYDROXYLASE

(30) Priority: 23.07.1993 IT MI931649
(43) Date of publication of application: 19.07.1995
(73) Proprietor: PHARMACIA & UPJOHN S.p.A., 20152 Milano (IT)
(72) Inventor: VARASI, Mario, I-20146 Milan (IT); GIORDANI, Antonio, I-27100 Pavia (IT); SPECIALE, Carmela, I-20014 Nerviano (IT); CINI, Massimo, I-20020 Lainate (IT); BIANCHETTI, Alberto, I-20122 Milan (IT)
(74) Representative: Ferrario, Vittorino
(86) International application number: EP9402280
(87) International publication number: WO9503271

(56) References cited:
- EP-A- 0 501 378
- WO-A-92/18003
- JOURNAL OF BIOCHEMISTRY, vol.86, no.5, 1979, TOKYO JP pages 1199 - 1209 K. TANIZAWA, K. SODA 'The mechanism of kynurenine'
- TETRAHEDRON LETTERS, vol.33, no.21, 1992, OXFORD GB pages 3003 - 3004 R. PELLICCIARI ET AL. 'Stereospecific Synthesis of the Enantiomers of Nicotinylalanine, a Neuroprotective Agent' cited in the application
- HELVETICA CHIMICA ACTA, vol.63, no.24, 1980, BASEL CH pages 250 - 254 W. KELLER-SCHIERLEIN, B. JOOS '24. Stoffwechselprodukte von Microorganismen'
- JOURNAL OF ORGANIC CHEMISTRY, vol.50, no.19, 1985, EASTON US pages 3619 - 3622 J. E. NORDLANDER ET AL. 'A Short Enantiospecific Synthesis of 2-Amino-6,7-d ihydoxy-1,2,3,4-tetrahydronaphthalene' cited in the application
- LIEBIGS ANNALEN DER CHEMIE, no.12, 1991, WEINHEIM DE pages 1365 - 1368 K. BURGER ET AL. 'Synthese von gamma-Oxo-alpha-aminosäure-Derivaten aus Asparaginsäure'
- JOURNAL OF MEDICINAL CHEMISTRY, vol.37, no.5, 4 March 1994, WASHINGTON US pages 647 - 655 R. PELLICCI ET AL.ARI 'Modulation of the Kynurenine Pathway in Search of New Neuroprotective Agents. Synthesis and Preliminary Evaluation of (m-Nitrobenzoyl)alanine, a Potent Inhibitor of Kynurenine-3-hydroxylase'

## Description

The present invention refers to the use in the prevention and/or treatment of neurodegenerative diseases, such as, for example, Huntington's chorea, Alzheimer's disease, Parkinson's disease, dementia caused by acquired immunodeficiency syndrome (AIDS), infarctual dementia, cerebral ischemia, cerebral hypoxia or epilepsy, of 2-amino-4-phenyl-4-oxo-butyric acid derivatives which act as inhibitors of kynureninase and/or kynurenine-3-hydroxylase, the enzymes which form part of the metabolic pathway of kynurenine.
A second object of this invention comprises new compounds, either as single enantiomers or as mixture of enantiomers, derived from 2-amino-4-phenyl-4-oxo-butyric acid, and their pharmaceutically acceptable salts, a process for their preparation, and pharmaceutical compositions containing them.
It is well known in the art that through the kynurenine pathway, tryptophan metabolism gives rise to the formation of quinolinic acid on the one side and kynurenic acid on the other, as shown in the following diagram: 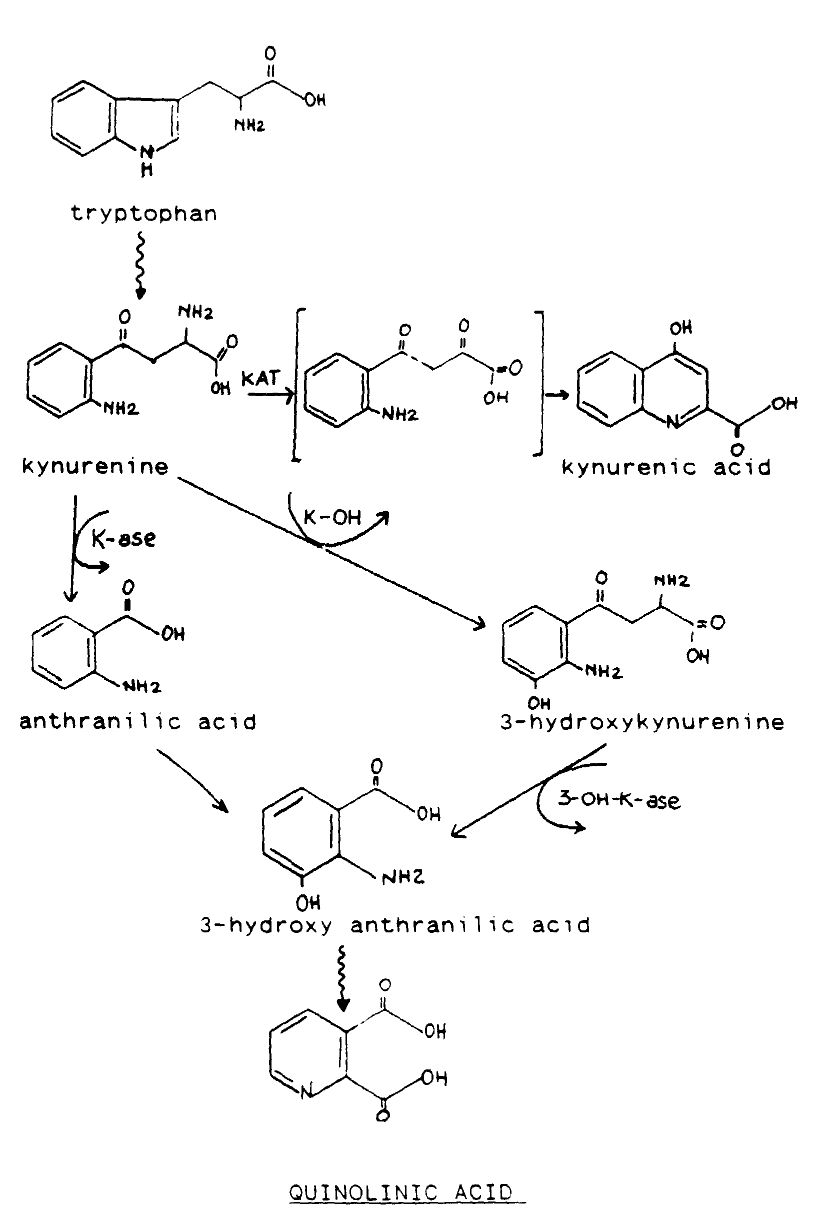 In the above diagram the symbol 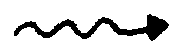 means that some steps of the tryptophan metabolism have been omitted.
- k-ase =: kynureninase enzyme
- K-OH =: kynurenine-3-hydroxylase enzyme
- 3-OH-K-ase =: 3-hydroxy kynureninase enzyme
- KAT =: kynurenine aminotransferase enzyme

Cerebral production of quinolinic acid, an endogenous agonist of the N-methyl-D-aspartic acid receptor, has been related to the pathogenesis of various neurodegenerative diseases [Life Science 35, 19-32 (1984)].
Direct infusion of quinolinic acid into the brain of laboratory animals produces specific lesions which, from a histopathologic viewpoint, closely resemble the damage observed in human neurodegenerative diseases such as, for example, Huntington's chorea and epilepsy of the temporal lobe [Science 219, 316-318 (1983)].
The metabolism of quinolinic acid has been studied in peripheral organs for many years [J. Biol. Chem. 238, 3369-3377 (1963); J. Biol. Chem. 239, 1208-1214 (1964)]. More recently, quinolinic acid has been identified in the rodent and human brain [Neurosc. Lett. 41, 247-252 (1983); Brain Res. 295, 352-356 (1984)], where the enzymes responsible for the metabolic pathway which leads to its synthesis are also present [J. Neurochem. 47, 23-30 (1986); J. Neurochem. 44, 446-454 (1985)].
An increase in the levels of quinolinic acid following transient ischemia in the gerbil has recently been reported [J. Neurochem. 60, 180-192 (1993)]. This phenomenon was associated with the induction of enzymes of the metabolic pathway of the kynurenines which lead to the formation of quinolinic acid.

An increase in the activity of the enzymes kynureninase and kynurenine-3-hydroxylase involved in its synthesis was simultaneously observed.

Consequently, compounds capable of inhibiting these enzymes, whose action would involve a decreased production of quinolinic acid (which can be considered as the neurotoxic product of tryptophan catabolism), would be useful in the prevention and treatment of all the pathologies involving quinolinic acid, or excessive activation of neurotransmission mediated by N-methyl-D-aspartic acid.

Object of this invention is the use in the prevention and/or treatment of neurodegenerative pathologies, such as, for example, Huntington's chorea, Alzheimer's disease, Parkinson's disease, dementia caused by acquired immunodeficiency syndrome (AIDS), multi-infarctual dementia, cerebral ischemia, cerebral hypoxia or epilepsy, of 2-amino-4-phenyl-4-oxo-butyric acid derivatives which act as kynureninase and/or kynurenine-3-hydroxylase enzyme inhibitors, having the following formula (I) 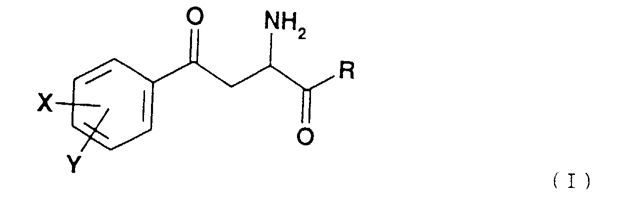 wherein
each of the groups X and Y is, independently, hydrogen, halogen, trifluoromethyl, hydroxy, C₁-C₆ alkyl, benzyl, C₆-C₁₀ aryl, -OR', -SR', 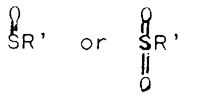 in which R' is C₁-C₆ alkyl or benzyl; and
R is hydroxy, amino, hydroxylamine, -OR', -NHR', 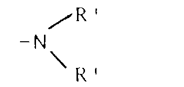 or -NHOR', wherein R' is as defined above.
This invention also comprises the pharmaceutically acceptable salts of the compounds of formula (I), as well as all the possible isomers (enantiomers) included in formula (I), both separately and in mixture, for use in the prevention and/or in the treatment of the above diseases.
EP-A-0501378 discloses and claims 4,6 disubstituted kynurenines as excitatory amino acid antagonists, characterized by an ortho-amino group on the phenyl ring. WO-A-9218003 describes and claims kynureninase inhibitors having formula KI 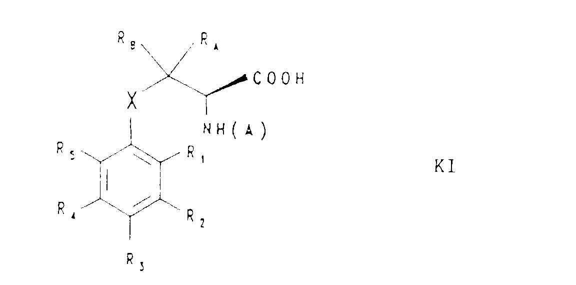 where X is CHOH, S, SO₂, SO, SONH, PO₂H or PONH₂; and R₁-R₅, A R_{A} and R_{B} are hydrogen or appropriate substituents.

This invention also refers to several derivatives of the aforementioned compounds of formula (I), as new compounds. These new compounds, which form a second object of the invention, are compounds having the following formula (IA) 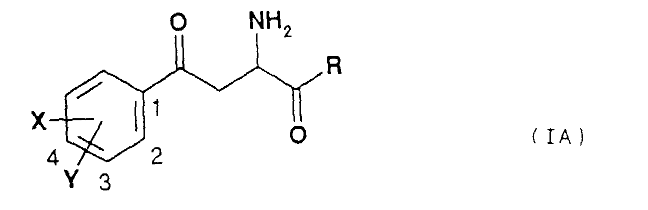 wherein
each of the groups X and Y is, independently, hydrogen, halogen, trifluoromethyl, hydroxy, C₁-C₆ alkyl, benzyl, C₆-C₁₀ aryl, OR', -SR', 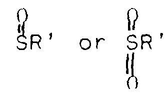 in which R' is C₁-C₆ alkyl or benzyl; and
R is hydroxy, -OR', amino, -NHR', 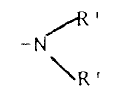 hydroxylamine or -NHOR', in which R' is as defined above;
provided that R is not hydroxy when:
(i) X and Y are simultaneously hydrogen; or
(ii) X and Y are in positions 3 and 4 of the phenyl ring and are simultaneously a hydroxy group, or a -OR' group in which R' is methyl; or
(iii) one of the X and Y groups is hydrogen and the other is in position 4 of the phenyl ring and is hydroxy, chlorine, fluorine, methyl, n-propyl, or methoxy,
and provided that R is not OR', in which R' is C₁-C₆ alkyl, when X and Y are both hydrogen or methoxy.

In its second object, this invention also comprises the pharmaceutically acceptable salts of the compounds of formula (IA), as well as all the possible isomers included in formula (IA), both separately and in mixture. The compounds of formula (IA) are a selected class of compounds of formula (I) and are thus also active in the prevention and/or treatment of all the diseases for which the compounds of formula (I) have been indicated as therapeutic agents.

With reference to both the previous formulae (I) and (IA), the meanings of the various substituents are as follows.

The term "C₁-C₆ alkyl" includes, for example, methyl, ethyl, n-propyl, isopropyl and n-butyl; preferably it represents methyl, ethyl or n-propyl.

The term "halogen" includes, chlorine, fluorine, iodine and bromine; preferably it represents chlorine, bromine or fluorine.

The term "C₆-C₁₀, aryl" includes, for example, phenyl and naphthyl; preferably it represents phenyl.

The compounds of formula (I) or (IA) have an asymmetric carbon atom and, for this reason, they can exist either as a mixture of optical isomers (enantiomeric mixture) or as a single optical isomer (enantiomer).

The pharmaceutically acceptable salts of the compounds of formula (I) or (IA) include both the salts of pharmaceutically acceptable acids, both inorganic acids, such as, e.g., hydrochloric, hydrobromic, nitric or sulphuric acid and organic acids such as, e.g., citric, tartaric, maleic, fumaric, methanesulfonic or ethanesulfonic acid; and the salts of pharmaceutically acceptable bases, both inorganic bases such as, e.g., hydroxides of alkali metals, for example, sodium or potassium, or alkaline-earth metals such as, e.g., calcium, magnesium, zinc or aluminium, and organic bases, such as, e.g., aliphatic amines such as, e.g., methylamine, diethylamine, trimethylamine, ethylamine or heterocyclic amines such as, e.g., piperidine.

A particular class of compounds of formula (IA) according to the invention are compounds of formula (IA) wherein R is hydroxy and wherein
(a) one of the groups X and Y is hydrogen and the other is C₁-C₆ alkyl or trifluoromethyl in position 2, 3 or 4 of the phenyl ring, provided that when the C₁-C₆ alkyl is in position 4 of the phenyl ring; it is, neither methyl nor n-propyl; or
(b) one of the groups X and Y is hydrogen and the other is a halogen atom in position 2, 3 or 4 of the phenyl ring, provided that when the halogen is in position 4 of the phenyl ring, it is neither chlorine nor fluorine; or
(c) one of the groups X and Y is hydrogen and the other is -OR' in which R' is C₁-C₆ alkyl, in position 2, 3 or 4 of the phenyl ring, provided that when -OR' is in position 4 of the phenyl ring, the C₁-C₆ alkyl is not methyl, or
(d) one of the groups X and Y is OR' in which R' is C₁-C₆ alkyl and the other is halogen;
either as single isomers or as mixture of isomers, and their pharmaceutically acceptable salts.

Specific examples of preferred compounds of formula (IA), either as single isomers or as mixture of isomers, are listed below:
2-amino-4-(2'-methoxyphenyl)-4-oxo-butyric acid;
2-amino-4-(3'-methoxyphenyl)-4-oxo-butyric acid;
2-amino-4-(2'-fluorophenyl)-4-oxo-butyric acid;
2-amino-4-(3'-fluorophenyl)-4-oxo-butyric acid;
2-amino-4-(2'-chlorophenyl)-4-oxo-butyric acid;
2-amino-4-(3'-chlorophenyl)-4-oxo-butyric acid;
2-amino-4-(3',4'-dichlorophenyl)-4-oxo-butyric acid;
2-amino-4-(2'-methylphenyl)-4-oxo-butyric acid;
2-amino-4-(2'-trifluoromethylphenyl)-4-oxo-butyric acid;
2-amino-4-(3'-trifluoromethylpnenyl)-4-oxo-butyric acid;
2-amino-4-(4'-trifluoromethylphenyl)-4-oxo-butyric acid;
2-amino-4-(2'-methoxy-5'-bromophenyl)-4-oxo-butyric acid;
2-amino-4-(2'-methoxy-5'-chlorophenyl)-4-oxo-butyric acid;
2-amino-4-(2'-methoxy-5'-fluorophenyl)-4-oxo-butyric acid;
and their pharmaceutically acceptable salts.

The new compounds of formula (IA) of the present invention may be prepared according to an analogy process comprising the following steps:
(A) reaction of a compound of formula (II) 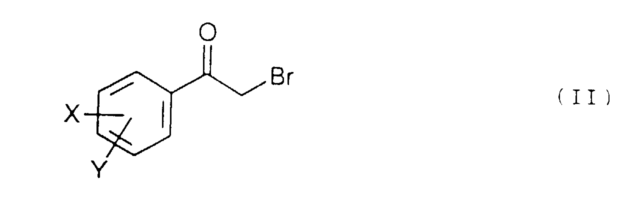 wherein X and Y are as defined above, with an alkalimetal or alkaline-earth metal salt of a compound of formula (III) 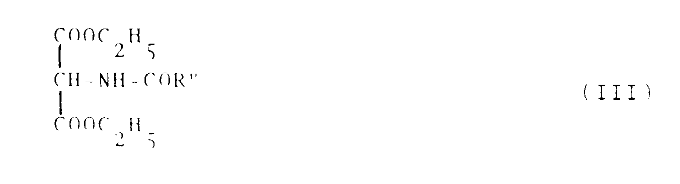 wherein R" is hydrogen or methyl, so obtaining a compound of formula (IV) 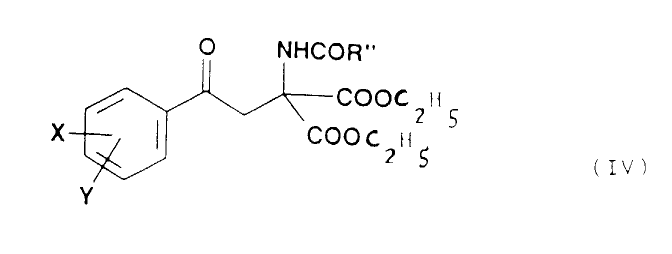 wherein X, Y and R" are as defined above;
(B) treatment of a compound of formula (IV) with concentrated halogenidric acid, so obtaining a compound of formula (IA) wherein X and Y are as defined above and R is hydroxy;
(C) optional conversion of a compound of formula (IA) into another compound of formula (IA) in which R is other than hydroxy;
(D) optional salification of a compound of formula (IA);
(E) optional separation of an isomeric mixture of a compound of formula (IA) into single isomers.

The compounds of formula (IA) can also be obtained directly as single optical isomers (enantiomers) by means of enantioselective synthesis.
The reaction of the step (A) can be carried out, for example, in the presence of a suitable solvent, such as, e.g., ethanol or dimethylformamide (DMF), at a temperature which may vary, for example, from about 0°C to about 80°C, for a period of time which may vary, for example, from about 4 to about 24 hours.
The reaction of the step (B) which causes hydrolysis and simultaneous decarboxylation of a compound of formula (IV) can be carried out, for example, by treating a compound of formula (IV) with a concentrated halogenidric acid, such as, e.g., 37% hydrochloric acid or 48% hydrobromic acid, for example, at a temperature of about 100°C for a period of, e.g., approximately 4-8 hours. The conversion of step (C) can be carried out with well known techniques, starting from compounds of formula (IA), wherein X and Y are as defined above and R is hydroxy.
Salification of step (D) can be carried out using conventional methods.
Separation at step (E) can be carried out according to techniques and procedures well known in the art; for example, chromatography on chiral stationary phases or resolution via diastereoisomeric salt formation and subsequent separation by selective crystallization. Separation by crystallization of diastereoisomer salts obtained by the salification of compounds of formula (IA) or appropriate protected derivatives thereof with suitable optically active acids or bases may be carried out using well known procedures normally used in the resolution of aminoacids into their enantiomers (for example: P. Newman, Optical Resolution Pr-ocedures for Chemical Compounds, Vol. 2, part 1, optical resolution information centre, Manhattan College, Riverdale, New York, 1981).
Protection at the acid moiety as well as the basic group of a compound of formula (IA) may be carried out by known methods. Suitable protecting groups for the carboxylic moiety are, e.g., methyl, ethyl, benzyl and tert-butyl esters, preferably benzyl and tert-butyl esters. Suitable protecting groups for the amino moiety are amides such as, e.g., acetylamide, trifluoroacetylamide or benzoyl amide, preferably acetylamide; or carbamates such as, e.g., tert-butoxycarbonylamino or benzyloxycarbonylamino, preferably benzyloxycarbonylamino.
The compounds of formula (II) are either known compounds, commercially available, or compounds that can be prepared through well known methods.
Also the compounds of formula (III) are either known compounds or may be obtained according to known methods from known compounds.

As already stated, compounds of formula (IA) may be also obtained by means of an enantioselective synthetic procedure using reactions known in the art. Enantiomers of compounds of formula (IA) may be prepared according to procedures well known by one of ordinary skill in the art (see, for example, F.G. Salituro, I.A. McDonald, J. Org. Chem, 53, 6138-39, 1988; R. Pellicciari, Tetrahedron Letters 33, 3003-3004, 1992). A general enantioselective synthetic procedure is summarized in Scheme I below where all substituents, unless otherwise stated, are as defined above and wherein Z is a suitable amino protecting group. 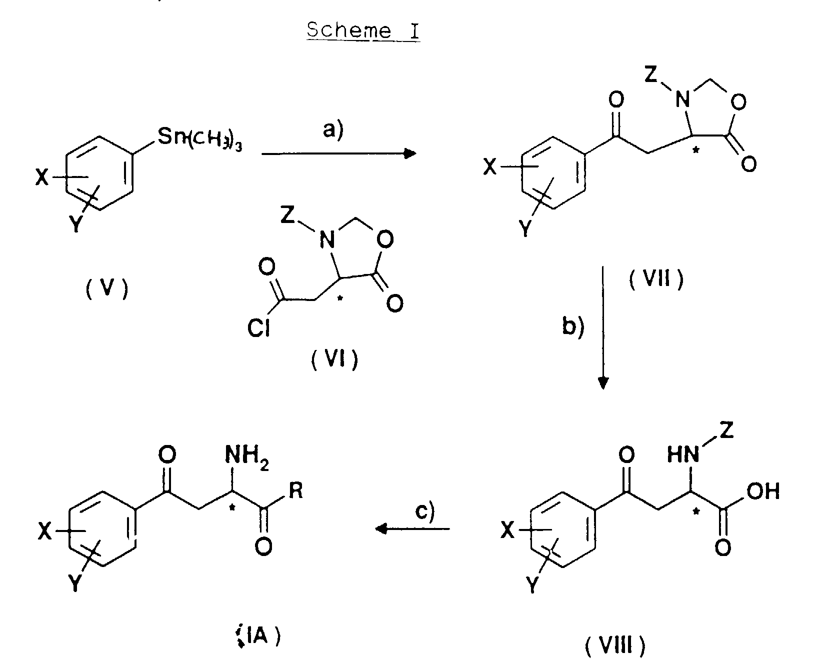 More in detail, a single (R) or (S) enantiomer of a compound of formula (IA) may be obtained by the process which comprises:
a) reacting a compound of formula (V) 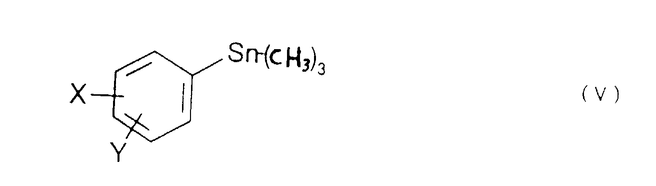 wherein
   X and Y are as defined above, with a single (R) or (S) enantiomer of a compound of formula (VI) 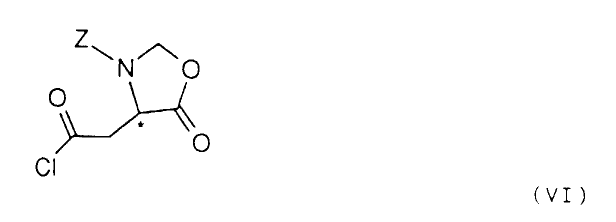 wherein
   Z is a suitable amino protecting group, so obtaining a single (R) or (S) enantiomer of a compound of formula (VII) 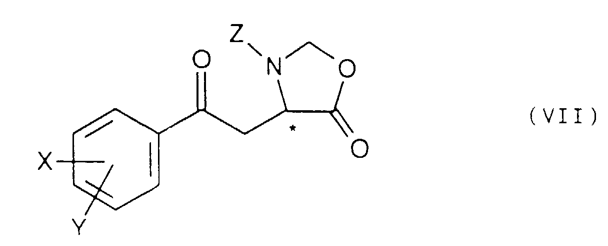 wherein
   X, Y and Z are as defined above;
b) deprotecting a compound of formula (VII) so obtaining a single (R) or (S) enantiomer of a compound of formula (VIII) 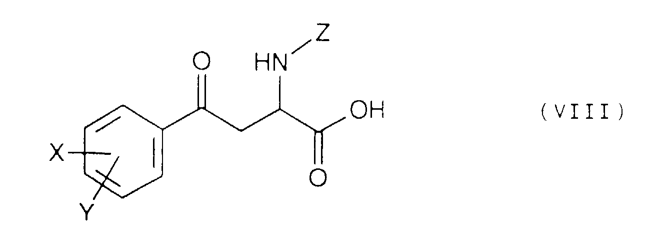 wherein
   X, Y and Z are as defined above; and
c) further deprotecting a compound of formula (VIII) so obtaining a single (R) or (S) enantiomer of a compound of formula (IA) which, depending on the reaction conditions, is a free amino acid or its salt; the (R) or (S) configuration of a compound of formula (VI) being retained throughout the whole process leading to the compounds of formula (IA).

The compounds of formula (IA) may be also obtained directly from a compound of formula (VII) following known procedures, e.g. acid hydrolysis.
Preferably, the suitable amino protecting group Z is a benzyloxycarbonyl group.
The reaction of a compound of formula (V) with a single (R) or (S) enantiomer of a compound of formula (VI), as source of the appropriate chirality, may be carried out, for example, in the presence of catalytic amounts of a soluble palladium catalyst, such as, e.g., bis(triphenylphosphine), palladium (II) dichloride, palladium (II) chloride diacetonitrile complex or bis(dibenzylidene acetone) palladium, in a suitable organic solvent such as, e.g., toluene, chloroform or tetrahydrofuran, at a temperature ranging from about 25°C to about 60°C, for a time ranging from about 1 hour to about 10 hours (see, for example, J. Org. Chem. 48, 4634-4642, 1983 and J. Am. Chem. Soc. 105 (19), 6129-6137, 1983), to obtain a compound of formula (VII) of appropriate (R) or (S) configuration, the same of the starting compound of formula (VI). In fact, as already said the (R) or (S) configuration of a compound of formula (VI) is retained throughout the whole process.
A compound of formula (VII) may be sequentially deprotected to the corresponding (R) or (S) enantiomer of a compound of formula (VIII) according to known methods (Chem. Pharm. Bull. 17(8), 1679-1686, 1969), for example, treating a compound of formula (VII) with a diluted aqueous alkali metal hydroxide such as, e.g., sodium, potassium or lithium hydroxide, preferably sodium hydroxide, in a suitable organic solvent such as, e.g., ethanol or methanol.
A compound of formula (VIII) may be further deprotected to the corresponding (R) or (S) enantiomer of formula (IA) according to known methods; for example, by reaction with trimethyl silyl iodide in a suitable organic solvent such as chloroform (see J. Chem. Soc. Comm. 495-496, 1979), or by catalytic transfer hydrogenation (see J. Org. Chem. 44, 3442-44, 1979 and J. Org. Chem. 43, 4194-96, 1978), or by acid hydrolysis, typically by warming a compound of formula (VIII) in 6N hydrochloric acid at a temperature ranging from about 60°C to about 110°C, for a time ranging from about 2 hours to about 10 hours.
The compounds of formula (IA) obtained according to the above procedures may be in the form of free amino acid or of its salts; the conversion of a salt to the corresponding free amino acid may be carried out, if desired, following known procedures; for example, by treating the appropriate salt of a compound of formula (IA) dissolved in a suitable solvent, typically isopropanol, with propylene oxide or using ion-exchange chromatography technique, or inducing the precipitation of the free amino acid from its aqueous solution at isoelectric point.
The compounds of formula (V) are known compounds (J. Chem. Soc. B, 1036-40, 1967 and J. Organometallic Chem. 10, 529-30, 1967) or may be prepared according to known methods either by direct organolithium transmetallation of the appropriate aromatics (J. Org. Chem. 41, 3653-3663, 1976; J. Org. Chem. 41, 1487-1493, 1976 and J. Organometallic Chem. 11, 209-16, 1968) or by metal halogen-exchange of a suitably substituted bromo or iodobenzene (R.G. Janes, Org. React. VI, 339-366), followed by reaction with trimethyl tin chloride in a suitable organic solvent such as, e.g., ethyl ether or tetrahydrofuran. The compounds of formula (V) may be also obtained following the procedures described in Bull. Chem. Soc. Japan 56, 3855-56, 1983, by Palladium catalyzed reaction of hexamethylditin with the appropriate aryl iodide.
The compounds of formula (VI) are known compounds or may be prepared according to known methods (Tetrahedron 42, 6551-54, 1986; Synthetic Communications 20 (22), 3507-3517, 1990).

Alternatively, the compounds of formula (IA) may be prepared, either as single enantiomers or as enantiomeric mixture, by a further procedure as outlined in Scheme II below, where all substituents, unless otherwise indicated, are as defined above: 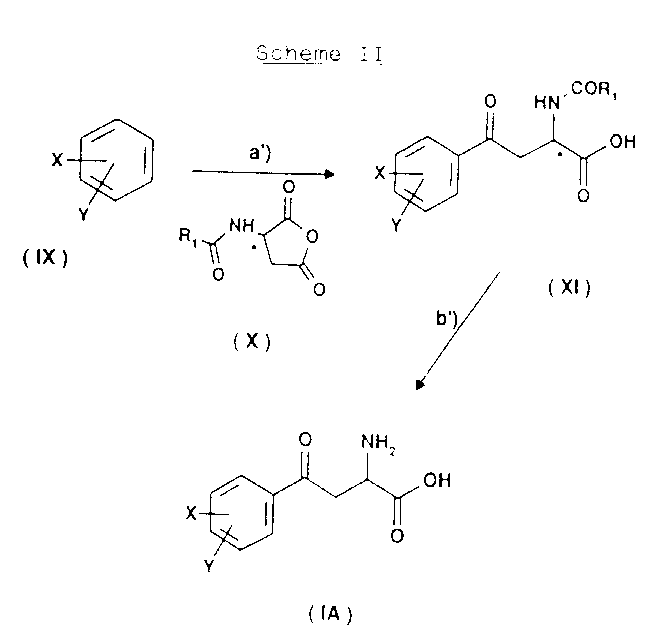 and wherein R₁' is hydrogen, methyl, trifluoromethyl, C₁-C₆ alkoxy or benzyloxy.
More in detail, a compound of formula (IA) either as a single (R) or (S) enantiomer or as a racemic mixture may be obtained following a process which comprises:
a') reacting a compound of formula (IX) 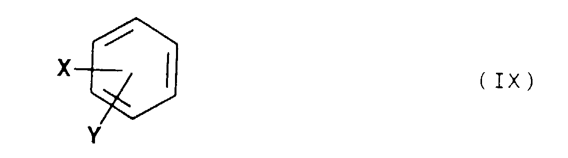 wherein
   X and Y are as defined above, with a compound of formula (X) 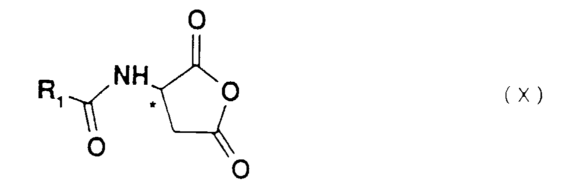 wherein
   R₁ is hydrogen, methyl, trifluoromethyl, C₁-C₆ alkoxy or benzyloxy, either as a single (R) or (S) enantiomer or as racemic mixture, so obtaining a compound of formula (XI) 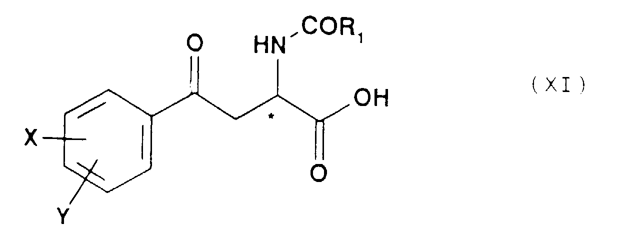 wherein
   X, Y and R₁ are as defined above, either as a single (R) or (S) enantiomer or as racemic mixture;
b') converting a compound of formula (XI) either as a single (R) or (S) enantiomer or as racemic mixture into a single (R) or (S) enantiomer or racemic mixture of a compound of formula (IA) wherein X and Y are as defined above and R is hydroxy, and, if desired, converting a compound of formula (IA) wherein R is hydroxy into compound of formula (IA) wherein R is other than hydroxy.

Preferably R₁ is trifluoromethyl, methoxy or ethoxy. The reaction of a compound of formula (IX) with a compound of formula (X) as described under step a') may be carried out according to known methods (see, for example, J.E. Nordlander, J. Org. Chem., 50, 3619-22, 1985 and D.G. Melillo, J. Org. Chem. 52, 5143-50, 1987); for example, the reaction may be performed in the presence of a suitable Lewis acid catalyst, in an inert solvent such as, e.g., dichloromethane or dichloroethane typically dichloromethane or in a suitable aromatic hydrocarbon such as, e.g., chlorobenzene, benzene, nitrobenzene or a mixture of such solvents, at a temperature ranging from about -5°C to about 60°C; optionally in the presence of a cosolvent such as, for example, nitromethane.
A suitable Lewis acid may be, e.g., anhydrous aluminium trichloride, anhydrous tin dichloride, titanium tetrachloride or zinc dichloride, typically aluminium trichloride.
The conversion of a compound of formula (XI) into a compound of formula (IA) as described under step b') may be carried out according to known procedures under either acidic or alkaline conditions.
Alkaline hydrolysis may be performed by an alkali metal hydroxide such as, e.g., lithium, sodium or potassium hydroxide or sodium carbonate, in a suitable solvent such as, e.g., aqueous methanol or ethanol, at a temperature ranging from about 0°C to about 50°C. Acid hydrolysis may be carried out by a halogenidric acid such as, e.g., hydrochloric or hydrobromic acid, at a temperature ranging from about 60° to about 110 C for a time which may vary from 4 hours to 12 hours.
The conversion of a compound of formula (IA) wherein R is hydroxy into compound of formula (IA) wherein R is other than hydroxy may be carried out following known procedures.
When a compound of formula (XI) is obtained as a mixture of regioisomers, the corresponding isomers may be separated and recovered by techniques known in the art such as chromatography or separation by selective crystallization.
The compounds of formula (IX) and (X) are known compounds or may be obtained by known procedures.
The other known compounds of formula (I) may also be prepared as above described for the new compounds of formula (IA).
The efficacy of the compounds of the invention in the inhibition of kynureninase has been evaluated in rat liver homogenates as described by Takikawa O. et al. in J. Biol. Chem. 261 (8), 3648-3652 (1986), with slight modifications.
HPLC method was essentially Takikawa's method, using the same Fluorimetric detection (ex. 313 nm, em. 420 nm) but changing the column (Nova-Pak C18 3.9 × 300 mm) and the mobile phase (phosphate buffer 80 mM, 13% CH₃CN pH 2.5).

The efficacy of the compounds of the invention in the inhibition of the enzyme kynurenine-3-hydroxylase has been evaluated in rat liver homogenate determining the conversion of L-kynurenine to L-3-hydroxy-kynurenine according to the method described below.

### Kynurenine-3-hydroxylase assay in the rat liver

Rat liver is homogenized in cold 0.32 M sucrose. The homogenates are centrifuged at 12000 × rpm for 30 minutes at 4°C. The precipitate, after having been washed three times with 0.32 M sucrose by means of centrifugation (12000 × rpm for 30 min), is resuspended in 20 mM K-buffer + 0.14 M KCl at pH 7 (1 g of liver in 6.5 ml).
The mixture of the reaction (200 µl) contains: 65 µl of resuspended homogenate. 50 mM phosphate buffer at pH 7.5, 2 mM MgCl₂, 1.5 mM glucose-6-phosphate, 4 U/ml glucose-6-phosphate dehydrogenase, 0.4 mM NADP and 25 µM kynurenine and the molecules to be tested at the screening dose of 1 mM and 100 µM.
The reaction at 37°C is terminated by the addition of 200 µl of 1 mM HClO₄ after 10 minutes of incubation. The concentrations of 3-hydroxy-kynurenine, produced in the absence or presence of the tested molecules are determined by HPLC with coulometric detection (pot. +0.20 V), using for the separation a reversed phase column C18, 10 cm long and a 3 µm particulate. The composition of the mobile phase was: 950 ml of water for HPLC, 20 ml of acetonitrile, 9 ml of triethylamine, 5.9 ml of phosphoric acid, 100 mg of Na EDTA and 1.5 g of heptanesulfonic acid.
The compounds of the present invention: (R,S) 2-amino-4-phenyl-4-oxo-butyric acid (FCE 27377) and (R,S) 2-amino-4-(2'-methoxyphenyl)-4-oxo-butyric acid (FCE 27384) have been tested according to the methods described above. The obtained results, which have been reported in the following Table 1, demonstrate the efficacy of the tested compounds in inhibiting the activity of kynureninase and/or kynurenine-3-hydroxylase in rat liver homogenates at the indicated concentrations.

**Table 1**

| % INHIBITION | | | | |
|---|---|---|---|---|
| Kynureninase | | | Kynurenine-3-hydroxylase | |
| | 100 µM | ¹ mM | 100 µM | 1 mM |
| FCE 27377 | 46 | 86 | 75 | 96 |
| FCE 27384 | 96 | 97 | 3 | 30 |
| 27377 = (R,S) 2-amino-4-phenyl-4-oxo-butyric acid | | | | |
| 27384 = (R,S) 2-amino-4-(2'-methoxyphenyl)-4-oxo-butyric acid | | | | |

The efficacy of the compounds according to the invention as kynureninase and/or kynurenine-3-hydroxylase inhibitors has also been evaluated in rat brain homogenates following the methods described below.

### Kynureninase assay in the rat brain

Partial purification of rat brain kynureninase was performed according to Lee et al., "Isolation and characterization of kynureninase from rat liver", advances in Tryptophan Research, 431-434, 1992. To perform rat brain kynureninase assay, a reaction mixture (final volume 0.2 ml) containing 100 mM tris-HCl, pH 8.0, 50 µl pyridoxal phosohate, 300 µM kynurenine, 20 µl of partially purified enzyme and 100 µM of inhibitor solution was prepared.
The reaction was carried out at 37°C for 3 hours and then stopped by adding 50 µl of perchloric acid 2N. After centrifugation at 11000 rpm for 15 min., anthranilic acid in the supernatant was determined fluorimetrically in a HPLC system (as described for the liver method).

### Kynurenine-3-hydroxylase assay in the rat brain

Kynurenine-3-hydroxylase activity was quantified by the conversion of L-kynurenine to 3-hydroxykynurenine.
Brain was homogenized in ice-cold 0.32 M sucrose and centrifugated at 12000 × g. for 30 min at 4°C. The pellet was washed three times with 0.32 M sucrose by centrifugation and suspended in 0.14 M KCl in 20 mM K-phosphate buffer pH 7 (1 g tissue in 2 ml buffer).
The reaction mixture contained: 75 µl of suspended homogenate; 100 µl of substrate solution containing 50 mM K-phosphate buffer pH 7.5, 2 mM MgCl₂, 0.4 mM NADPH and 50 µM L-kynurenine (final concentration); 25 µl of different concentrations of inhibitor solutions.
The reaction was stopped by addition of 200 µl of 1 M HClO₄ after 60 min. incubation.
3-hydroxykynurenine formed was quantified by HPLC with coulometric detection (working voltage was + 0.2 V). The column was a 10 cm C18 reversed phase (3 µm). The mobile phase consisted of 950 ml distilled water, 20 ml acetonitrile, 9 ml triethylamine, 5.9 ml phosphoric acid, 100 mg sodium EDTA and 1.5 g heptanesulfonic acid. The flow-rate was 1 ml/min.
A representative number of compounds of the present invention:
FCE 27377 (R,S)-2-amino-4-phenyl-4-oxo-butyric acid;
FCE 28468 (S)-2-amino-4-phenyl-4-oxo-butyric acid;
FCE 28469 (R)-2-amino-4-phenyl-4-oxo-butyric acid;
FCE 27384 (R,S)-2-amino-4-(2'-methoxyphenyl)-4-oxo-butyric acid;
FCE 28631 (R,S)-2-amino-4-(2'-fluorophenyl)-4-oxo-butyric acid;
FCE 28628 (R,S)-2-amino-4-(4'-methoxyphenyl)-4-oxo-butyric acid;
FCE 28630 (R,S)-2-amino-4-(2'-methylphenyl)-4-oxo-butyric acid;
FCE 28626 (S)-2-amino-4-(2'-methoxyphenyl)-4-oxo-butyric acid;
FCE 28629 (R,S)-2-amino-4-(3'-methoxyphenyl)-4-oxo-butyric acid;
FCE 28680 (R,S)-2-amino-4-(3'-trifluoromethylphenyl)-4-oxo-butyric acid;
FCE 28751 (R,S)-2-amino-4-(4'-chlorophenyl)-4-oxo-butyric acid;
FCE 28752 (R,S)-2-amino-4-(3'-chlorophenyl)-4-oxo-butyric acid:
FCE 28753 (R,S)-2-amino-4-(2'-chloroohenyl)-4-oxo-butyric acid;
FCE 28764 (R,S)-2-amino-4-(3'-fluorophenyl)-4-oxo-butyric acid;
FCE 28766 (R,S)-2-amino-4-(2'-methoxy-5'-fluoropheryl)-4-oxo-butyric acid;
FCE 28833 (R,S)-2-amino-4-(3',4'-dichlorophenyl)-4-oxo-butyric acid; and
FCE 28836 (R,S)-2-amino-4-(2'-methoxy-5'-chlorophenyl-4-oxo-butyric acid;
have been tested according to the methods in rat brain homogenates described above.
The obtained results reported in the following Table 2 show the efficacy of the tested compounds in inhibiting the activity of kynureninase and/or kynurenine-3-hydroxylase enzymes in rat brain homogenates.
The activity of the tested compounds has been expressed as percentage of enzyme inhibition at a concentration of 100 µM and, where evaluated, as IC₅₀ (concentration which inhibits 50% of the enzyme activity).

**Table 2**

| Compound | % INHIBITION AT 100 µM | |
|---|---|---|
| | Kyn-Ase | Kyn-3-OH-Ase |
| FCE 27377 | 35.6 | 71 (IC₅₀ = 42 µM) |
| FCE 28468 | 61 | 85 (IC₅₀ = 16 µM) |
| FCE 28469 | 19 | 19 |
| FCE 27384 | 86 (IC₅₀ = 6 µM) | 4 |
| FCE 28631 | 52 | 47 |
| FCE 28628 | 17 | 79 (IC₅₀ = 30 µM) |
| FCE 28630 | 40 | 81 (IC₅₀ = 23 µM) |
| FCE 28626 | 84 (IC₅₀ = 5 µM) | 3 |
| FCE 28629 | 54 | 29 |
| FCE 28680 | 67 | 17 |
| FCE 28751 | 0 | 85 (IC₅₀ = 7 µM) |
| FCE 28752 | 60 | 93 (IC₅₀ =0.4 µM) |
| FCE 28753 | 71 | 22 |
| FCE 28764 | 0 | 93 (IC₅₀ =0.9 µM) |
| FCE 28766 | 81 | 2 |
| FCE 28833 | nc | 99 (IC₅₀ =0.2 µM) |
| FCE 28836 | nc | 73 (IC₅₀ =33 µM) |
| nc = not calculated. | | |

The compounds of this invention can be administered to a mammalian such as a human, in a variety of dosage forms, e.g. orally, in the form of tablets, capsules, sugar or film-coated tablets, liquid solutions or suspensions; rectally in the form of suppositories; parenterally, e.g., intramuscularly or by intravenous injection or infusion.
The dosage depends on age, weight, conditions of the patient and administration route; for example, dosage adapted to oral administration in adults can range from approximately 25 to 500 mg per dose, 1 to 5 times per day.
This invention includes pharmaceutical compositions comprising a compound of the invention in combination with a pharmaceutically acceptable excipient (which may be a carrier or a diluent).
The pharmaceutical compositions containing the compounds of this invention are generally prepared according to conventional methodologies and are administered in a suitable pharmaceutical form.
For example, oral solid forms may contain the active ingredient together with diluents, such as, e.g., lactose, dextrose, sucrose, cellulose, corn starch or potato starch; lubricants such as, e.g., silica, talc, stearic acid, magnesium or calcium stearate and/or polyethylene glycols; binders such as, e.g., starches, gum arabic, gelatine, methylcellulose, carboxymethylcellulose or polyvinylpyrrolidone; desaggregating agents such as, e.g., starches, alginic acid, alginates or sodium starch glycolate; effervescent mixtures; dyestuffs, sweeteners; wetting agents, such as, e.g., lecithin, polysorbate, laurylsulphates; and in general, non toxic and pharmacologically inactive substances used in pharmaceutical formulations.
Said pharmaceutical preparations may be manufactured in the known manner, for example by means of mixing, granulating, tabletting, sugar-coating or film-coating processes. The liquid dispersions for oral administration may be, for instance, syrups, emulsions and suspensions. The syrups may contain as carrier, for example, saccharose or saccharose with glycerine and/or mannitol and/or sorbitol; in particular, a syrup which should be administered to diabetic patients may contain as carriers only products which do not metabolize to glucose or which metabolize in very small quantities to glucose, for example sorbitol.
The suspensions and the emulsions may contain as carrier for example, a natural gum, agar, sodium alginate, pectin, methylcellulose, carboxymethylcellulose or polyvinyl alcohol.
The suspensions or solutions for intramuscular injections may contain together with the active compound a pharmaceutically acceptable carrier, such as, e.g., sterile water, olive oil, ethyl oleate or glycols, such as, e.g., propylene glycol, and if desired, a suitable amount of lidocaine hydrochloride.
The solutions for intravenous injection or infusion may contain as carrier, for example, sterile water or preferably they may be in the form of sterile aqueous isotonic saline solutions.
The suppositories may contain together with the active compound a pharmaceutically acceptable carrier, e.g. cocoa-butter, polyethylene glycol, a polyoxyethylene sorbitan acid ester surfactant or lecithin.

The following examples illustrate but do not limit the invention.

### Example 1

### Preparation of the ethyl 4-(2'-methoxyphenyl)-4-oxo-2-formyl-amide-2-carbethoxybutyrate.

Add the diethyl α-formamido malonate, 2.85 g (0.014 moles) to a sodium ethylate solution obtained from 0.32 g (0.014 moles) of metal sodium and 30 ml of absolute ethanol and leave to stir for 45 minutes at 40-50°C. Bring the solution obtained to room temperature and drop in a solution of α-bromo-2'-methoxyacetophenone, 3 g (0.0131 moles) in 10 ml of absolute ethanol. Leave to stir at room temperature for 24 hours. The reaction mixture is evaporated to dryness, diluted with ethyl acetate and washed with water. The organic phase is separated, dried (Na₂SO₄), filtered and evaporated to give 5 g of a dark brown oil which solidifies when left on its own. The solid obtained is triturated with ethyl ether and filtered to give 2.8 g of the desired product as a white solid.
m.p. = 110-112°C.

| | | | |
|---|---|---|---|
| Calculated = | C 58.11 | H 6.02 | N 3.99 |
| Found = | C 58.01 | H 6.06 | N 3.94. |

The following compounds can be prepared by proceeding in the same way:
ethyl 4-phenyl-4-oxo-2-formylamido-2-carbethoxybutyrate; m.p. 110°-111°C;
ethyl 4-(3'-methoxyphenyl)-4-oxo-2-formylamido-2-carbethoxybutyrate;
ethyl 4-(4'-methoxyphenyl)-4-oxo-2-formylamido-2-carbethoxybutyrate;
ethyl 4-(2'-fluorophenyl)-4-oxo-2-formylamido-2-carbethoxybutyrate;
ethyl 4-(3'-fluorophenyl)-4-oxo-2-formylamido-2-carbethoxybutyrate;
ethyl 4-(4'-fluorophenyl)-4-oxo-2-formylamido-2-carbethoxybutyrate;
ethyl 4-(2'-chlorophenyl)-4-oxo-2-formylamido-2-carbethoxy butyrate;
ethyl 4-(3'-chlorophenyl)-4-oxo-2-formylamido-2-carbethoxy butyrate;
ethyl 4-(4'-chlorophenyl)-4-oxo-2-formylamido-2-carbethoxy butyrate;
ethyl 4-(2'-bromophenyl)-4-oxo-2-formylamido-2-carbethoxy butyrate;
ethyl 4-(3'-bromophenyl)-4-oxo-2-formylamido-2-carbethoxy butyrate;
ethyl 4-(4'-bromophenyl)-4-oxo-2-formylamido-2-carbethoxy butyrate;
ethyl 4-(2'-methylphenyl)-4-oxo-2-formylamido-2-carbethoxy butyrate;
ethyl 4-(3'-methylphenyl)-4-oxo-2-formylamido-2-carbethoxy butyrate;
ethyl 4-(4'-methylphenyl)-4-oxo-2-formylamido-2-carbethoxy butyrate;
ethyl 4-(2'-trifluoromethylphenyl)-4-oxo-2-formylamido-2-carbethoxy butyrate;
ethyl 4-(3'-trifluoromethylphenyl)-4-oxo-2-formylamido-2-carbethoxy butyrate;
ethyl 4-(4'-trifluoromethylphenyl )-4-oxo-2-formylamido-2-carbethoxy butyrate;
ethyl 4-(2' ,4'-dichlorophenyl)-4-oxo-2-formylamido-2-carbethoxy butyrate;
ethyl 4-(2' ,5'-dichlorophenyl)-4-oxo-2-formylamido-2-carbethoxy butyrate;
ethyl 4-(3' ,4'-dichlorophenyl)-4-oxo-2-formylamido-2-carbethoxy butyrate;
ethyl 4-(2',4'-dimethoxyphenyl)-4-oxo-2-formylamido-2-carbethoxy butyrate;
ethyl 4-(2',5'-dimethoxyphenyl)-4-oxo-2-formylamido-2-carbethoxy butyrate;
ethyl 4-(3' ,4'-dimethoxyphenyl)-4-oxo-2-formylamido-2-carbethoxy butyrate;
ethyl 4-(3',5'-dimethoxyphenyl)-4-oxo-2-formylamido-2-carbethoxy butyrate;
ethyl 4-(2'-methoxy-4'-chlorophenyl)-4-oxo-2-formylamido-2-carbethoxy butyrate; and
ethyl 4-(2'-methoxy-4'-bromophenyl)-4-oxo-2-formylamido-2-carbethoxy butyrate.

### Example 2

The below listed compounds may be prepared following the procedure of Example 1 using diethyl acetamido malonate instead of diethylformamido malonate:
Ethyl 4-(3'-methoxyphenyl)-4-oxo-2-acetamido-2-carboethoxy-butyrate
obtained in 43% yield as colourless plates, m.p. 119-120°C
- MS (EI; 70 eV):: 365 (M⁺⁰, 23), 320 (5), 292 (54), 250 (38), 233 (23), 204 (12), 135 (100).
- ¹H-NMR (200 MHz; CDCl₃) ppm:: 1.12 (6H, t), 1.98 (3H, s), 3.85 (3H, s), 4.28 (4H, q), 7.10-7.60 (4H, m).

| | | | | |
|---|---|---|---|---|
| C₁₈H₂₃NO₇ | Calculated | C 59.18; | H 6.35; | N 3.84 |
| | Found | C 59.16; | H 6.42; | N 3.76. |

Ethyl 4-(4'-methoxyphenyl)-4-oxo-2-acetamido-2-carboethoxy-butyrate
obtained in 78% yield as colourless needles, m.p. 120-122°C.
- MS (EI; 70 eV):: 365 (M⁺⁰, 12), 320 (2), 292 (54), 261 (8), 250 (30), 204 (9), 135 (100).
- ¹H-NMR (80 MHz, CDCl₃) ppm:: 1.21 (6H, t), 1.95 (3H, s), 3.90 (3H, s), 4.25 (2H, s), 4.28 (4H, q), 6.89 (2H, d), 7.10 (1H, broad s), 7.90 (2H, d).

| | | | | |
|---|---|---|---|---|
| C₁₈H₂₃NO₇ | Calculated | C 59.17; | H 6.30; | N 3.83 |
| | Found | C 59.16; | H 6.40; | N 3.80. |

Ethyl 4-(2'-fluorophenyl)-4-oxo-2-acetamido-2-carboethoxy-butyrate
obtained in 72% yield as colourless prisms m.p. 79-81.5°C
- MS (EI, 70 eV):: 353 (M⁺⁰, 5), 280 (19), 238 (38), 192 (21), 123 (100).
- ¹H-NMR (80 MHz; CDCl₃) ppm:: 1.20 (6H, t), 1.98 (3H, s), 4.25 (4H, q), 4.30 (2H, s), 7.0-7.7 (3H, m), 7.88 (1H, dd).

| | | | | |
|---|---|---|---|---|
| C₁₇H₂₀FNO₆ | Calculated | C 57.84; | H 5.71; | N 3.96 |
| | Found | C 56.36; | H 5.99; | N 3.98. |

Ethyl 4-(3'-fluorophenyl)-4-oxo-2-acetamido-2-carboethoxy-butyrate
obtained in 48% yield as colourless plates m.p. 121-123°C.
- MS (EI; eV):: 354 (M⁺⁰, 22), 280 (9), 238 (23), 123 (100).
- H¹-NMR (200 MHz, CDCl₃) ppm:: 1.20 (6H, t), 1.96 (3H, s), 4.25 (2H, s), 4.28 (4H, q), 7.20 (1H, broad s), 7.28 (1H, m), 7.46 (1H, m), 7.62 (1H, dd), 7.88 (1H, dd).

| | | | | |
|---|---|---|---|---|
| C₁₇H₂₀FNO₆ | Calculated | C 57.84; | H 5.71; | N 3.96 |
| | Found | C 57.88; | H 5.86; | N 3.82. |

Ethyl 4-(2'-chlorophenyl)-4-oxo-2-acetamido-2-carboethoxy-butyrate
obtained in 37% yield as colourless needles, m.p. 157-158°C.
- MS (EI; 70 eV) m/z:: 369 (M⁺⁰, 1.8), 296 (13), 254 (20), 208 (5.4), 139 (100).
- H¹-NMR (200 MHz, CDCl₃) ppm:: 1.27 (6H, t), 2.0 (3H, s), 4.25 (2H, s), 4.32 (4H, q), 7.12 (1H, broad s), 7.24-7.57 (4H, m).

| | | | | | |
|---|---|---|---|---|---|
| C₁₇H₂₀ClNO₆ | Calculated | C 55.26; | H 5.46; | N 3.79; | Cl 9.60 |
| | Found | C 55.39; | H 5.57; | N 3.82; | Cl 9.40. |

Ethyl 4-(3'-chlorophenyl)-4-oxo-2-acetamido-2-carboethoxy-butyrate
obtained in 58% yield as colourless prisms, m.p. 84-86°C.

Ethyl 4-(2'-methylphenyl)-4-oxo-2-acetamido-2-carboethoxy-butyrate
obtained in 55% yield as slight yellow plates, m.p. 146-147°C.
- MS (EI; 70 eV) m/z:: 349 (M⁺⁰, 10.7), 304 (2.3), 276 (19), 234 (27), 119 (100).
- ¹H-NMR (80 MHz, CDCl₃) ppm:: 1.25 (6H, t), 2.0 (3H, s), 2.46 (3H, s), 4.22 (2H, s), 4.29 (4H, q), 7.1-7.6 (m, 4H), 7.8 (1H, dd).

| | | | | |
|---|---|---|---|---|
| C₁₈H₂₃NO₆ | Calculated | C 61.94; | H 6.64; | N 4.01 |
| | Found | C 61.80; | H 6.72; | N 4.03. |

Ethyl 4-(2'-trifluoromethylphenyl)-4-oxo-2-acetamido-2-carboethoxy-butyrate
obtained in 37% yield as colourless prisms, m.p. 104°-105°C
- MS (EI; 70 eV) m/z:: 403 (M⁺⁰; 3.8), 358 (2.3), 330 (16), 288 (23), 242 (8). 173 (100)
- ¹H-NMR (80 MHz, CDCl₃) ppm:: 1.28 (6H, t), 2.0 (3H, s), 4.22 (2H, s), 4.28 (4H, q), 7.15 (1H, broad s), 7.5-7.9 (4H, m)

| | | | | |
|---|---|---|---|---|
| C₁₈H₂₀F₃NO₆ | Calculated | C 53.59; | H 5.00; | N 3.47 |
| | Found | C 53.72; | H 5.19; | N 3.38. |

Ethyl 4-(3'-trifluoromethylphenyl)-4-oxo-2-acetamido-2-carboethoxy-butyrate
obtained in 42% yield as light yellow needles, m.p. 91-92°C.
- H¹-NMR (80 MHz, CDCl₃) ppm:: 1.23 (6H, t), 1.98 (3H, s), 4.30 (2H, s), 4.25 (4H, q), 7.12 (1H, broad s), 7.5-8.3 (4H, m).

| | | | | |
|---|---|---|---|---|
| C₁₈H₂₀F₃NO₆ | Calculated | C 53.59; | H 5.00; | N 3.47 |
| | Found | C 53.62; | H 5.09; | N 3.38. |

Ethyl 4-(4'-trifluoromethylphenyl)-4-oxo-2-acetamido-2-carboethoxy-butyrate
obtained in 24% yield as yellow oil.
- H¹-NMR (80 MHz, CDCl₃) ppm:: 1.28 (6H, t), 1.98 (3H, s), 4.40 (2H, s), 4.28 (4H, q), 7.20 (1H, broad s), 8.05 (2H, d), 8.35 (2H, d).

Ethyl 4-(2'-methoxy-5'-fluorophenyl)-4-oxo-2-acetamido-2-carboethoxy-butyrate
obtained in 64% yield as colourless needles, m.p. 123-124°C.
- MS (EI; 70 eV) m/z:: 383 (M⁺⁰, 2.5), 310 (12.5), 268 (13), 153 (100)
- H¹-NMR (200 MHz, CDCl₃) ppm:: 1.24 (6H, t), 1.98 (3H, s), 3.93 (3H, s), 4.34 (2H, s), 4.25 (4H, q), 6.90 (1H, dd), 7.10 (1H, broad s), 7.20 (1H, m), 7.40 (1H, dd)

| | | | | |
|---|---|---|---|---|
| C₁₈H₂₂FNO₇ | Calculated | C 56.45; | N 5.79; | N 3.65 |
| | Found | C 56.60; | H 5.84; | H 3.66. |

Ethyl 4-(3',4'-dichlorophenyl)-4-oxo-2-acetamido-2-carboethoxy-butyrate
obtained in 42% yield as yellow oil.
- ¹H-NMR (80 MHz, CDCl₃) ppm:: 1.20 (6H, t), 1.98 (3H, s), 4.20 (2H, s), 4.25 (4H, q), 7.10 (1H, broad s), 7.48 (1H, d), 7.75 (1H, dd), 7.88 (1H, d).

### Example 3

### Preparation of (R,S)-2-amino-4-(2'-methoxyphenyl)-4-oxo-butyric acid.HCl.

To 1 g (0.0028 moles) of ethyl 4-(2'-methoxyphenyl)-4-oxo-2-formylamido-2-carbethoxybutyrate add 3 ml of glacial acetic acid and 10 ml of 37% HCl. Keep at reflux for 8 hours. The solution is then evaporated to dryness; the residue is taken up three times with water and the solution re-evaporated. The residue obtained after this treatment is dissolved again in water and the aqueous solution washed with dichloromethane. The aqueous phase is then evaporated to dryness to give 0.67 g of the desired product as a white solid.
m.p. 130° dec.

| | | | | |
|---|---|---|---|---|
| Calculated = | C 50.87 | H 5.43 | N 5.39 | Cl 13.65 |
| Found = | C 50.74 | H 5.68 | N 5.43 | Cl 13.35 |

The following compounds can be prepared by proceeding in the same way:
(R,S)-2-amino-4-phenyl-4-oxo-butyric acid.HCl m.p. 170°C dec.

(R,S)-2-amino-4-(3'-methoxyphenyl)-4-oxo-butyric acid.HCl obtained in 73% yield as colourless prisms m.p. 115°C dec.
MS (FAB⁺) m/z: 224 (MH⁺)
H¹-NMR (200 MHz, DMSOd₆) ppm: 3.70 (2H, d), 3.86 (3H, s), 4.33 (1H, m), 7.25 (1H, dd), 7.40-7.60 (3H, m), 8.50 (3H, broad s)

| | | | | | |
|---|---|---|---|---|---|
| C₁₁H₁₃NO₄.HCl.H₂O | Calc. | C 47.56; | H 5.81; | N 5.04; | Cl 12.79 |
| | Found | C 47.60; | H 5.83; | N 5.00; | Cl 12.81 |

(R,S)-2-amino-4-(4'-methoxyphenyl)-4-oxo-butyric acid.HCl obtained in 69% yield as colourless needles. m.p. 160°C dec.
- MS (FAB⁺) m/z:: 224 (MH⁺)
- H¹-NMR (200 MHz, DMSOd₆) ppm:: 3.63 (2H, d), 3.84 (3H, s), 4.28 (1H, m), 7.03 (2H, d), 7.92 (2H, d), 8.43 (3H broad s).
C₁₁H₁₃NO₄.HCl Calculated: C 50.87; H 5.43; N 5.39; Cl 13.65
Found: C 51.87; H 5.53; N 5.22; Cl 13.63.

(R,S)-2-amino-4-(2'-fluorophenyl)-4-oxo-butyric acid.HCl obtained in 78% yield as colourless prisms, m.p.213°-214°C
- MS (FAB⁺) m/z:: 212 (MH⁺)
- H¹-NMR (200 MHz, DMSOd₆) ppm:: 3.65 (2H, m), 4.52 (1H, t), 7.38 (2H, m), 7.73 (1H, m), 7.85 (1H, m), 8.50 (3H, broad s).
C₁₀H₁₁FClNO₃ Calculated:C 48.52; H 4.48; N 5.65; Cl 14.34
Found: C 48.47; H 4.52; N 5.61; Cl 14.40.

(R,S)-2-amino-4-(3'-fluorophenyl)-4-oxo-butyric acid.HCl obtained in 75% yield, as colourless prisms, m.p. 194-195°C dec.
MS (FAB⁻) m/z: 246 (55), 210 [(M-H)⁻, 100], 193 (57)
(FAB⁺) m/z: 212 (M+H)⁺
¹H-NMR (200 MHz, DMSOd₆) ppm: 3.77 (2H, m), 4.32 (1H, dd), 7.50-7.70 (2H, m), 7.76 (1H, m), 7.85 (1H, m), 8.59 (3H, broad s), 13.80 (1H, broad s)

| | | | | | |
|---|---|---|---|---|---|
| C₁₀H₁₁FClNO₃ | Calc. | C 48.52; | H 4.48; | N 5.65; | Cl 14.34 |
| | Found | C 48.50; | H 4.55; | N 5.55; | Cl 14.09 |

(R,S)-2-amino-4-(2'-chlorophenyl)-4-oxo-butyric acid. HCl obtained in 83% yield as colourless needles, m.p. 187-188°C dec.
MS (FAB⁺) m/z: 228 (M+H)⁺
¹H-NMR (200 MHz, DMSOd₆) ppm: 3.68 (2H, dd), 4.30 (1H, t), 7.44-7.60 (3H, m), 7.78 (1H, dd), 8.74 (3H, broad s).

| | | | | | |
|---|---|---|---|---|---|
| C₁₀H₁₁Cl₂NO₃ | Calculated | C 45.49; | H 4.20; | N 5.30; | Cl 26.89 |
| | Found | C 45.54; | H 4.26; | N 5.30; | Cl 26.76 |

(R,S)-2-amino-4-(3'-chlorophenyl)-4-oxo-butyric acid.HCl obtained in 51% yield as colourless plates, m.p. 198-200°C dec.
MS (FAB⁺) m/z: 455 (84), 228 (M+H⁺, 100)
H¹-NMR (200 MHz, DMSOd₆) ppm: 3.67 (2H, d), 4.23 (1H, t), 7.60 (1H, t), 7.72, (1H, dd), 7.91 (2H, m), 8.50 (3H, broad s).

| | | | | | |
|---|---|---|---|---|---|
| C₁₀H₁₁Cl₂NO₃ | Calculated | C 45.49; | H 4.20; | N 5.30; | Cl 26.89 |
| | Found | C 45.54; | H 4.28; | N 5.35; | Cl 26.70 |

(R,S)-2-amino-4-(4'-chlorophenyl)-4-oxo-butyric acid.HCl obtained in 79% yield as colourless prisms, m.p. 204-206°C
MS (FAB⁺) m/z: 455 (53.8), 228 (M+H⁺, 100)
H¹-NMR (200 MHz, DMSOd₆) ppm: 3.75 (2H, d), 4.30 (1H, t), 7.65 (2H, d), 7.98 (2H, d), 8.60 (3H, broad s).

| | | | | | |
|---|---|---|---|---|---|
| C₁₀H₁₁Cl₂NO₃ | Calculated | C 45.49; | H 4.20; | H 5.30; | Cl 26.89 |
| | Found | C 45.64; | H 4.24; | H 5.23; | Cl 26.57 |

(R,S)-2-amino-4-(2'-bromophenyl)-4-oxo-butyric acid.HCl
(R,S)-2-amino-4-(3'-bromophenyl)-4-oxo-butyric acid.HCl
(R,S)-2-amino-4-(4'-bromophenyl)-4-oxo-butyric acid.HCl
(R,S)-2-amino-4-(4'-fluorophenyl)-4-oxo-butyric acid.HCl

(R,S)-2-amino-4-(2'-methylphenyl)-4-oxo-butyric acid.HCl obtained in 96% yield as colourless prisms, m.p. 192-193°C dec.
MS (FAB⁺) m/z: 415 (4), 316 (2), 208 (M+H⁺, 100)
¹H-NMR (200 MHz, DMSOd₆) ppm: 2.42 (3H, s), 3.63 (2H, d), 2.28 (1H, t), 7.35 (2H, t), 7.52 (1H, b), 7.81 (1H, d), 8.60 (1H, broad s).

| | | | | | |
|---|---|---|---|---|---|
| C₁₁H₁₄ClNO₃ | Calculated | C 54.26; | H 5.79; | N 5.75; | Cl 14.56 |
| | Found | C 54.06; | H 5.83; | N 5.76; | Cl 14.83 |

(R,S)-2-amino-4-(3'-methylphenyl)-4-oxo-butyric acid.HCl

(R,S)-2-amino-4-(4'-methylphenyl)-4-oxo-butyric acid.HCl

(R,S)-2-amino-4-(2'-trifluoromethylphenyl)-4-oxo-butyric acid.HCl
obtained in 86% yield as colourless plates, m.p. 170°C dec.
MS (FAB⁺) m/z: 523 (6), 262 (M+H⁺, 100)
(FAB⁻) m/z: 296 (63), 260 ([M-H)⁻, 100), 243 (61)
¹H-NMR (200 MHz, DMSOd₆) ppm: 3.58 (1H, dd), 3.72 (1H, dd), 4.52 (1H, t), 7.70-7.90 (4H, m), 8.60 (3H, broads s).

| | | | | | |
|---|---|---|---|---|---|
| C₁₁H₁₁F₃ClNO₃ | Calculated | C 44.37; | H 3.72; | N 4.70; | Cl 11.93 |
| | Found | C 44.29; | H 3.77; | N 4.69; | Cl 12.08 |

(R,S)-2-amino-4-(3'-trifluoromethylphenyl)-4-oxo-butyric acid.HCl
obtained in 93% yield as colourless plates, m.p. 164-165°C dec.
MS (FAB⁺) m/z: 262 (M+H⁺)
(FAB⁻) m/z: 296 (42), 260 ([M-H]⁻, 100), 243 (65).
¹H-NMR (200 MHz, DMSOd₆) ppm: 3.82 (2H, d), 4.37 (1H, t), 7.80 (1H, t), 8.08 (1H, d), 8.26 (1H, s), 8.28 (1H, d), 8.60 (3H, broad s).

| | | | | | |
|---|---|---|---|---|---|
| C₁₁H₁₁F₃ClNO₃ | Calculated | C 44.37; | H 3.72; | N 4.70; | Cl 11.93 |
| | Found | C 44.59; | H 3.94; | N 4.56; | Cl 11.86 |

(R,S)-2-amino-4-(4'-trifluoromethylphenyl)-4-oxo-butyric acid.HCl
obtained in 56% yield as colourless plates, m.p. 193-195°C dec.
MS (FAB⁺) m/z: 262 (M+H⁺)
¹H-NMR (200 MHz, DMSOd₆) ppm: 3.80 (2H; m), 4.35 (1H, d), 7.95 (2H, d), 8.19 (2H, d), 8.51 (3H, broad s), 13.80 (1H, broad s)

| | | | | | |
|---|---|---|---|---|---|
| C₁₁H₁₁F₃ClNO₃ | Calculated | C 44.37; | H 3.72; | N 4.70; | Cl 11.93 |
| | Found | C 43.42; | H 3.75; | N 4.57; | Cl 11.54 |

(R,S)-2-amino-4-(2'-4'-dichlorophenyl)-4-oxo-butyric acid.HCl
(R,S)-2-amino-4-(2'-5'-dichlorophenyl)-4-oxo-butyric acid.HCl
(R,S)-2-amino-4-(2',4'-dimethoxyphenyl)-4-oxo-butyric acid.HCl
(R,S)-2-amino-4-(2',5'-dimethoxyphenyl)-4-oxo-butyric acid.HCl
(R,S)-2-amino-4-(3',4'-dimethoxyphenyl)-4-oxo-butyric acid.HCl
(R,S)-2-amino-4-(3',5'-dimethoxyphenyl)-4-oxo-butyric acid.HCl
(R,S)-2-amino-4-(2'-methoxy-5'-bromophenyl)-4-oxo-butyric acid.HCl
(R,S)-2-amino-4-(2'-methoxy-5'-fluorophenyl)-4-oxo-butyric acid.HCl
obtained in 83% yield as colourless prisms, m.p. 123-124°C dec.
MS (FAB⁺) m/z: 483 (7), 242 (M+H⁺, 100)
(FAB⁻) m/z: 276 (100), 240 ([M-H], 82), 223 (38)
¹H-NMR (200 MHz, DMSOd₆) ppm: 3.63 (2H, m), 3.90 (3H, s), 4.62 (2H, dd), 7.26 (1H, dd), 7.35-7.55 (2H, m), 8.50 (3H, broad s), 13.85 (1H, broad s).

| | | | | | |
|---|---|---|---|---|---|
| C₁₁H₁₃FClNO₄ | Calculated | C 47.61; | H 4.72; | N 5.04; | Cl 12.79 |
| | Found | C 47.23; | H 4.75; | N 4.95; | Cl 12.62 |

(R,S)-2-amino-4-(3',4'-dichlorophenyl)-4-oxo-butyric acid.HCl
obtained in 74% yield as colourless prisms, m.p. 212-213°C dec.

| | | | | | |
|---|---|---|---|---|---|
| C₁₀H₁₀Cl₃NO₃ | Calculated | C 40.23; | H 3.37; | N 4.69; | Cl 35.68 |
| | Found | C 40.14; | H 3.45; | N 4.53; | Cl 34.27 |

- ¹H-NMR (200 MHz, DMSOd₆) ppm:: 3.80 (2H, d), 4.28 (1H, t), 7.83 (1H, d), 7.98 (1H, d), 8.20 (1H, s), 8.50 (3H broad s)
- MS (FAB⁺) m/z:: 262 (M+H)

### Example 4

### Preparation of 4-(S)-[2-(2'-methoxyphenyl)-2-oxo-ethyl]-5-oxo-3-benzyloxycarbonyl-oxazolidine

Mix (S)-3-(benzyloxycarbonyl )-5-oxo-4-oxazolidine acetyl chloride (10 g, 34 mmol) and dry toluene (150 ml) at room temperature. Add, under dry nitrogen atmosphere, (2'-methoxyphenyl)trimethyl tin (10 g, 37 mmol), followed by bis(triphenylphosphine) palladium (II) dichloride (50 mg, 0.07 mmol).
Heat under stirring 8 hours, cool and wash the organic phase with saturated sodium hydrogen carbonate solution (3 × 50 ml), dry (Na₂SO₄) and evaporate the solvent in vacuo.
Purify by silica gel flash chromatography (8 × 24 cm; hexane/ethyl ether 50%) to give the title compound as a colourless oil (4.9 g; 38%).
[α]$\frac{\text{25°C}}{\text{D}}$ = + 45.5° (C = 1.4; methanol)
- ¹H-NMR (200 MHz, DMSOd₆) ppm:: 3.48 (1H, dd), 3.80 (3H, s), 3.91 (1H, dd), 4.58 (1H, t), 5.12 (2H, m), 5.28 (1H, d), 5.48 (1H, d), 7.02-7.60 (9H, m).
- MS (EI; 70 eV) m/z:: 369 (M⁺⁰, 13); 278 (46), 234 (23), 135 (100), 91 (81).

Analogously, starting from (R)-3-(benzyloxycarbonyl)-5-oxo-4-oxazolidine acetyl chloride, the corresponding 4-(R)-[2-(2'-methoxyphenyl)-2-oxo-ethyl]-5-oxo-3-benzyloxycarbonyl-oxazolidine [α]$\frac{\text{25°C}}{\text{D}}$ = + 47.1° (C = 1.4; methanol) may be prepared.

### Example 5

### Preparation of (S)-2-(N-benzyloxycarbonyl amino)-4-oxo-4-(2'-methoxyphenyl)butyric acid.

Dissolve 4-(S)-[2-(2'-methoxyphenyl)-2-oxo-ethyl]-5-oxo-3-benzyloxycarbonyl-oxazolidine (4.6 g, 18 mmol) in 95% ethanol (60 ml), cool to 0°C and add under stirring 1N sodium hydroxide (14 ml); stir for one hour at 0°C and five hours at room temperature.
Pour the resulting suspension into 2N hydrochloric acid (60 ml) at 0°C, then add water (60 ml) and extract with ethyl acetate (3 × 80 ml), dry (Na₂SO₄) and evaporate the solvent in vacuo.
Purify by silica gel flash chromatography (4 × 12 cm; CH₂Cl₂/ethanol 2%) to obtain the title compound (2.8 g; 63%) as a colourless oil.
[α]$\frac{\text{25°C}}{\text{D}}$ = + 15.36 (C = 1.9; abs. EtOH).
- ¹H-NMR (200 MHz; CDCl₃):: 3.55 (1H, dd), 3.82 (1H, dd), 3.90 (3H, s), 4.78 (1H, m), 5.10 (2H, s), 5.90 (1H, d), 6.98-7.51 (8H, m), 7.83 (1H, dd).
- MS (FAB⁺) m/z:: 358 (M+H⁺, 61), 340 (30), 314 (58), 135 (100).

Analogously, starting from 4-(R)-[2-(2'-methoxyphenyl)-2-oxo-ethyl]-5-oxo-3-benzyloxycarbonyl-oxazolidine, the corresponding (R)-2-(N-benzyloxycarbonylamino)-4-oxo-4-(2'-methoxyphenyl) butyric acid [α]$\frac{\text{25°C}}{\text{D}}$ = - 16.56° (C = 1.1; abs. EtOH) may be prepared.

### Example 6

### Preparation of (S)-2-amino-4-(2'-methoxyphenyl)-4-oxo-butyric acid.HCl

Add (S)-2-(N-carbobenzyloxy amino)-4-oxo-4-(2'-methoxyphenyl)butanoic acid (1.8 g, 5 mmol), dissolved into glacial acetic acid (10 ml), to 6N hydrochloric acid (60 ml) and warm the resulting solution at 70°C for 12 hours. Cool and wash the obtained solution with ethyl ether (2 × 20 ml), then evaporate the aqueous phase in vacuo to obtain a colourless solid which is recrystallized from ethanol/ethyl acetate to give the title compound (0.8 g; 60%) as colourless prisms, m.p. 130°C dec.
[α]$\frac{\text{25°C}}{\text{D}}$ = + 36.8° (HCl 6N, C = 0.34)
MS (FAB⁺) m/z: 224 (M+H⁺; 10G), 178 (11), 151 (38)
¹H-NMR (200 MHz, DMSOd₆) ppm: 3.60 (2H, d), 3.90 (3H, s), 4.26 (1H, m), 7.0-7.70 (4H, m), 8.40 (3H, broad s), 13.80 (1H, broad s).

| | | | | | |
|---|---|---|---|---|---|
| C₁₁H₁₄ClNO₄ | Calculated | C 50.87; | H 5.43; | N 5.39; | Cl 13.65 |
| | Found | C 50.12; | H 5.42; | N 5.28; | Cl 13.55 |

Analogously, starting from (R)-2-(N-benzyloxycarbonylamino)-4-oxo-4-(2'-methoxyphenyl)butyric acid the corresponding
(R)-2-amino-4-(2'-methoxyphenyl)-4-oxo-butyric acid.HCl [α]$\frac{\text{25°C}}{\text{D}}$ = - 39.86° (HCl 6H, C = 0.34)

| | | | | | |
|---|---|---|---|---|---|
| C₁₁H₁₄ClNO₄ | Calculated | C 50.87; | H 5.43; | N 5.39; | Cl 13.65 |
| | Found | C 49.03; | H 5.70; | N 5.12; | Cl 13.75 |

may be prepared.

### Example 7

### Preparation of (S)-2-(N-methoxycarbonyl amino)-4-oxo-4-phenyl butyric acid.

To a suspension of anhydrous aluminium chloride (11.5 g, 87 mmol) in dry dichloromethane (70 ml), cooled at 0°C, add nitromethane (5 ml, 87 mmol) on stirring, and under dry nitrogen atmosphere, followed by dry benzene (30 ml), then warm to room temperature and stir for one hour. Add S-(N-methoxycarbonyl)aspartic anhydride (6 g, 35 mmol) portionwise, at room temperature and bubbling dry nitrogen through the solution.
Stir the obtained solution at room temperature for 4 hours, then warm to 40°C for 10 hours. Cool the reaction mixture and pour it into 37% hydrochloric acid/ice (100 ml/100 g), dilute with dichloromethane (100 ml) and wash the organic phase with 2N hydrochloric acid (3 × 50 ml). Evaporate the solvent in vacuo and take up the residue in ethyl ether (80 ml), extract with saturated sodium hydrogen carbonate solution (3 × 30 ml), wash with ethyl ether (2 × 10 ml), then treat with 37% hydrochloric acid at 0°C, on stirring, until pH is 3, extract the resulting suspension with ethyl acetate (3 × 30 ml) and dry (Na₂SO₄).
Evaporate the solvent in vacuo to obtain the title compound (5.5 g, 63%) as a colourless oil.
[α]$\frac{\text{25°C}}{\text{D}}$ = + 97.06° (C = 1.35, chloroform)
- MS (FAB⁺) m/z:: 252 (M+H⁺, 100), 234 (45), 206 (50)
- ¹H-NMR (200 MHz, DMSOd₆) ppm:: 3.56 (1H, dd), 3.68 (3H, s), 3.78 (1H, dd), 4.75 (1H, m), 5.84 (1H, d), 6.10 (1H, broad s), 7.4-7.68 (3H, m), 7.91 (2H, dd).

Analogously, starting from (R)-N-methoxycarbonyl aspartic anhydride:
R-(2)-(N-methoxycarbonylamino)-4-oxo-4-phenyl-butyric acid
[α]$\frac{\text{25°C}}{\text{D}}$ = - 92.7° (C = 1.38, chloroform) may be prepared.

### Example 8

### Preparation of (S)-2-amino-4-oxo-4-phenyl-butyric acid.HCl

Dissolve(S)-2-(N-methoxycarbonyl amino)-4-oxo-4-phenyl-butyric acid (1.5 g, 6 mmol) into glacial acetic acid (10 ml) and add the resulting solution to 6N hydrochloric acid (50 ml).
Warm at 90°C 48 hours, then cool and evaporate in vacuo. Crystallize the obtained solid from absolute ethanol/ethyl acetate to obtain the title compound (0.93 g; 72%) as colourless prisms, m.p. 190°C dec.
[α]$\frac{\text{25°C}}{\text{D}}$ = + 41.4°C (C = 0.3; HCl 6N)
MS (FAB⁺) m/z: 194 (M+H⁺, 100).

| | | | | | |
|---|---|---|---|---|---|
| C₁₀H₁₂ClNO₃ | Calculated | C 52.33; | H 5.23; | N 6.10; | Cl 15.47 |
| | Found | C 51.10; | H 5.29; | N 6.18; | Cl 15.70 |

Analogously, starting from (R)-2-(N-methoxycarbonylamino)-4-oxo-4-phenyl-butyric acid:
(R)-2-amino-4-oxo-4-phenyl-butyric acid.HCl
[α]$\frac{\text{25°C}}{\text{D}}$ = + 42.8°C (C = 0.2; 6N HCl)

| | | | | | |
|---|---|---|---|---|---|
| C₁₀H₁₂ClNO₃ | Calculated | C 52.33; | H 5.23; | N 6.10; | Cl 15.47 |
| | Found | C 51.15; | H 5.29; | N 6.31; | Cl 15.70 |

may be prepared.

### Example 9

HPLC on chiral phase of (R,S)-2-amino-4-oxo-4-phenyl-butyric acid.HCl and (R,S)-amino-4-(2'-methoxyphenyl-4-oxo-butyric acid.HCl.
Using CROWNPAK CR(+) (15 cm × 4 mm) (Daicel) at a temperature of 30°C (± 1°C), and using aqueous HClO₄ (pH=2.0) as eluent at a flow of 0.8 ml/min, the above enantiomers (S)-2-amino-4-oxo-4-phenyl butyric acid.HCl and (R)-2-amino-4-oxo-4-phenyl butyric acid.HCl are separated and detected (UV 210 nm) by injecting their aqueous solution at a concentration of 230 mcg/ml (≅ 10⁻³ M), retention time: 11.82 min (R)-isomer, and 20.24 min (S)-isomer.
Analogously enantiomers of 2-amino-4-(2'-methoxyphenyl)-4-oxo butyric acid.HCl are separate using the same column, operating at a temperature of 25°C (± 1°C), eluting with aqueous HClO₄ (pH=2.5) at a flow of 1.0 ml/min, and injecting their solution of 260 mcg/ml in HClO₄ (pH=2.0), with UV detection at 210 nm.
Retention time:
(R)-2-amino-4-(2'-methoxyphenyl )-4-oxo-butyric acid ; 15.49 min and
(S)-2-amino-4-(2'-methoxyphenyl)-4-oxo-butyric acid:22.25 min.

### Example 10

### Preparation of (R,S)-methyl-2-(N-trifluoroacetamido)-4-oxo-4-(2'-methoxy-5'-chlorophenyl)butanoate.

To a suspension of anhydrous aluminium chloride (18 g, 135 mmol) in dry dichloromethane (250 ml), cooled at 0°C, add nitromethane (10 ml) on stirring, followed by 4-chloroanisole (11 ml, 90 mmol), then warm to room temperature and stir under dry nitrogen atmosphere for 1 hour.
Add (R,S)-N-trifluoroacetyl aspartic anhydride (20 g, 90 mmol) portionwise, at room temperature, under dry nitrogen atmosphere, and stir the reaction mixture for two hours, then warm at 40°C for 10 hours.
Cool the reaction mixture at room temperature and pour into 37% hydrochloric acid/ice (200 ml/200 g), dilute with dichloromethane (200 ml), and wash the organic layer with 2N hydrochloric acid (3 × 50 ml).
Evaporate the solvent in vacuo and take the residue up with ethyl ether (100 ml), extract with saturated sodium hydrogen carbonate solution (3 × 50 ml), wash the collected aqueous layers with ethyl ether (2 × 20 ml), then treat with 37% hydrochloric acid, under vigorous stirring at 0°C, the aqueous phase until the pH is 5. Extract the resulting suspension with ethyl acetate (4 × 40 ml) and dry (Na₂SO₄).
Evaporate the solvent in vacuo to obtain a yellow oil (14 g), then dissolve it in acetone (150 ml) and add, on stirring at room temperature, anhydrous potassium carbonate (28 g, 0.2 mcl), then treat with methyl iodide (12 ml, 0.2 mcl).
Stir the reaction mixture for 6 hours at room temperature, then filter the inorganic salts and evaporate the solvent in vacuo.
Take the resulting oil up in ethyl acetate (100 ml) and wash it with saturated sodium hydrogen carbonate solution (2 × 20 ml), dry the organic phase with Na₂SO₄ and evaporate the solvent in vacuo to obtain the pure title compound as a light yellow solid (12.8 g; 88%), m.p. 76-77°C.
- MS (EI; 70 eV) m/z:: 367 (M⁺, 10), 335 (7), 308 (2), 254(4), 169 (100).
- ¹H-NMR (200 MHz; CDCl₃) ppm:: 3.56 (1H, dd), 3.85 (1H, dd), 3.76 (3H, s), 3.92 (3H, s), 4.90 (1H, m), 6.93 (1H, d), 7.48 (1H, dd), 7.50 (1H, d).

### Example 11

### Preparation of (R,S)-2-amino-4-oxo-4-(2'-methoxy-5'-chlorophenyl)butyric acid

Dissolve (R,S) methyl-2-(N-trifluoroacetamide)-4-oxo-4-(2'-methoxy-5'-chlorophenyl)butyrate (8 g, 23 mmol) in 95% ethanol (180 ml), cool the resulting solution at 0°C, then treat with 1N sodium hydroxide (70 ml).
Stir at 0°C for one hour and at room temperature further 3 hours, then cool at 0°C and add 2N hydrochloric acid until pH is 6, the pure title compound precipitate on standing and cooling at 0°C, to give 3 g (51%) of colourless prisms, m.p. 147-148°C
¹H-NMR (200 MHz, DMSOd₆ + CF₃COOD) ppm: 3.58 (2H, d), 3.94 (3H, s), 4.35 (1H, t), 7.28 (1H, d), 7.62 (1H, dd), 7.71 (1H, d).

| | | | | | |
|---|---|---|---|---|---|
| C₁₁H₁₂ClNO₄ | Calculated | C 51.31; | H 4.70; | N 5.44; | Cl 13.77 |
| | Found | C 49.92; | H 4.98; | N 4,98; | Cl 12.85 |

Hydrochloride: m.p. 212-13°C
¹H-NMR (200 MHz, DMSOd₆) ppm: 3.60 (2H, d), 3.98 (3H, s), 4.27 (1H, t), 7.25 (1H, d), 7.60-7.80 (m, 2H), 8.40 (3H broad s).
MS (FAB⁺): m/z 258 [M⁺ H⁺]

### Example 12

### Preparation of (R,S)-2-[trifluoroacetamido)-4-oxo-4-(4'-fluorophenyl)butyric acid.

To a suspension of anhydrous aluminium chloride (23 g, 0.17 mcl) in dry dichloromethane (400 ml), cooled at 0°C, add nitromethane (20 ml), on stirring and under dry nitrogen atmosphere, followed by 4-fluorobenzene (13 ml, 0.14 mcl), then warm to room temperature and stir for an hour.
Add (R,S)-N-trifluoroacetyl aspartic anhydride (26.5 g, 0.12 mcl) portionwise at room temperature, then stir the resulting solution at room temperature for 2 hours. Warm the reaction mixture at 40°C for 20 hours, on stirring and under dry nitrogen atmosphere.
Cool the reaction mixture and pour it into 37% hydrochloric acid/ice (200 ml/200 g), dilute with dichloromethane (200 ml), wash the organic layer with 2N hydrochloric acid (2 × 50 ml).
Evaporate the solvent in vacuo and take the residue up with ethyl ether (150 ml), extract the organic phase with saturated sodium hydrogen carbonate solution (3 × 50 ml), collect and cool at 0°C the aqueous layers, then treat this aqueous solution, on cooling at 0°C and stirring, with 37% hydrochloric acid to precipitate at pH 5 the title compound.
Recrystallization from ethyl ether/hexane provides the pure title compound (19 g, 52%) as colourless prisms, m.p. 153-54°C.
- MS (FAB⁻):: 306([M-H]⁻, 79) , 193 (100)
- ¹H-NMR (200 MHz, DMSOd₆) ppm:: 3.58 (2H, d), 4.79 (1H, q), 7.35 (m, 2H), 8.03 (2H, m), 9.70 (1H, d)

### Example 13

### Preparation of (R,S)-2-amino-4-oxo-4-(4'-fluorophenyl)-butyric acid.

Dissolve 2-N-trifluoroacetamido-4-oxo-(4'-fluorophenyl) butyric acid (4 g, 13 mmol) in 95% ethanol (100 ml), cool the resulting solution at 0°C, then treat with 1N sodium hydroxide (29 ml).
Stir at 0°C for one hour, then at room temperature further 3 hours.
Cool the reaction mixture at 0°C and add 2N hydrochloric acid until pH is 6, the title compound crystallize on standing as cream prisms (2.2 g, 81%), m.p. 200°-201°C. ¹H-NMR (80 MHz, DMSOd₆ + CF₃COOD) ppm: 3.65 (2H, d), 4.36 (1H, t), 7.30 (2H, t), 8.08 (2H, dd)

| | | | | |
|---|---|---|---|---|
| C₁₀H₁₀FNO₃ | Calculated | C 56.92; | H 4.78: | N 6.64 |
| | Found | C 56.27: | H 4.86; | N 6.52 |

- Hydrochloride:: m.p. 181-82°C
- ¹H-NMR (200 MHz, DMSOd₆) ppm:: 3.78 (2H, d), 4.30 (1H, t), 7.40 (2H, t), 8.10 (2H, dd), 8.50 (3H, broad s).
- MS (FAB⁺):: m/z 212 (100), 123 (45)
C₁₀H₁₁FCNO₃ Calculated:C 48.50; H 4.48; N 5.65; Cl 14.31
Found: C 48.07; H 4.47; N 5.53; Cl 14.62.

### Example 14

Resolution of (R,S)-2-(N-benzyloxycarbonyl)-4-oxo-4-(2'-methoxyphenyl) butyric acid into its enantiomers by crystallization of its diastereoisomeric salt of ephedrine.

Dissolve(R,S)-2-(N-benzyloxycarbonylamino)-4-oxo-4-(2'-methoxyphenyl)butyric acid (0.61 g, 1.7 mmoles) in dry ethyl ether (10 ml), warm the obtained solution at 30°C and add a solution obtained dissolving (-)-Ephedrine (0.300 g, 1.8 mmoles) in dry ethyl ether (25 ml), warm the resulting suspension for 5 min, then cool to room temperature.
Filter the precipitate salt and wash it twice with dry ethyl ether to obtain the ephedrine salt as colourless powder (0.87 g,98%). [α]$\frac{\text{25°C}}{\text{D}}$=-16.25°C (C=0.5, abs.EtOH). Dissolve the obtained salt into ethyl acetate (40 ml), at reflux temperature, by adding isopropanol (2 ml), then cool the obtained solution at room temperature. After standing at room temperature 72 hours, 0.192 g (44%) of colourless crystals ([α]$\frac{\text{25°C}}{\text{D}}$ =-30.56°C (C=0.5, abs.EtOH); m.p. 159-161°C)) are obtained. The mother liquor is evaporated in vacuo to provide a colourless amorphous solid (0.66 g) named solid B.
Dissolve the above crystals in warm ethyl acetate (14 ml) and add absolute ethanol (5.5 ml), warm the suspension to reflux for 5 minutes to complete dissolution, then cool to room temperature.
After standing at room temperature 16 hours. 0.113 g of colourless crystals ([α]$\frac{\text{25°C}}{\text{D}}$ = -32.0 (C=0.65, abs.EtOH)) ; m.p. 163°-65°C are separated.
Dissolve the above crystals (0.100 g) in warm ethanol (2 ml) then cool the obtained solution at room temperature. After standing at room temperature 16 hours 0.085 g (20%) of colourless needles ([α]$\frac{\text{25°C}}{\text{D}}$ = -39.9°C (C=0.1, abs. EtOH); m.p. 166-67°C)) are separated.
¹H-NMR (80 MHz; DMSOd₆) ppm: 0.8 (3H, d), 2.5 (3H, s), 3.0-3.4 (3H, m), 3.85 (3H, s), 4.25 (1H, q), 5.0 (2H, s), 5.05 (1H, d), 6.9-7.6 (14H, m).

| | | | | |
|---|---|---|---|---|
| C₂₉H₃₄N₂O₇ | Calculated | C 66.70; | H 6.56; | N 5.36 |
| | Found | C 66.52; | H 6.64; | N 5.28 |

Dissolve the above salt (0.070 g, 0.13 mmol) in 2N hydrochloric acid (12 ml), and extract with ethyl acetate (4 × 10 ml), wash the collected organic extracts with 0.5N hydrochloric acid (2 × 3 ml), and dry (Na₂SO₄), evaporate the solvent to dryness in vacuo to obtain (S)-(+)-2-(N-carbobenzyloxy amino)-4-(2'-methoxyphenyl)-4-oxo-butyric acid (40 mg) as a colourless oil ([α]$\frac{\text{25°C}}{\text{D}}$ = + 17.82 (C=0.4, abs.EtOH)).
- H¹-NMR (80 MHz; CDCl₃) ppm:: 3.65 (2H, d), 3.90 (3H, s), 4.45 (1H, m), 5.20 (2H, s), 6.0 (1H, d), 7.0 (2H, m), 7.35 (5H, s), 7.50 (1H, m), 7.85 (1H, dd), 9.50 (1H, broad s).
Dissolve the above solid B (0.600 g, 1.1 mmol) into 2N hydrochloric acid (20 ml) and extract with ethyl acetate (4 × 10 ml), wash the collected organic extracts with 0.5N hydrochloric acid (2 × 5 ml), dry (Na₂SO₄) and evaporate to dryness to obtain 0.350 g (0.98 mmol) of enriched (R)-2-(N-carbobenzyloxy amino)-4-(2'-methoxyphenyl)-4-oxo-butyric acid.
Dissolve the above acid into warm ethyl acetate (25 ml), then add under stirring (+)-ephedrine (165 mg, 1 mmol), warm at 60°C for 10 min, then cool to room temperature. After standing at room temperature 16 hours 0.274 g of colourless needles ([α]$\frac{\text{25°C}}{\text{D}}$ = + 33.6°C (C=0.5, EtOH)), m.p. 165-66°C, are separated.
Dissolve the above crystals in absolute ethanol (3 ml) on warming at reflux, then cool at +5°C, on standing 12 hours, 0.103 g of colourless needles are separated ([α]$\frac{\text{25°C}}{\text{D}}$ = + 37.2°C (C=0.1, abs. EtOH)), m.p. 165-66°C.

### Example 15

Capsule, each weighing 0.230 g and containing 50 mg of the active substance can be prepared as follows: Composition for 500 capsules:

| | |
|---|---|
| (R,S)-2-amino-4-(2'-methoxyphenyl)-4-oxo-butyric acid | 25 g |
| Lactose | 80 g |
| Corn starch | 5 g |
| Magnesium stearate | 5 g |

This formulation can be incapsulated in two hard gelatin capsules of two pieces each with each capsule weighing 0.230 g.

### Example 16

### Intramuscular injection of 50 mg/ml

A pharmaceutical injectable composition can be manufactured dissolving 50 g of (R,S)-2-amino-4-(2'-methoxyphenyl)-4-oxo-butyric acid. HCl in sterile propyleneglycol (1000 ml) and sealed in 1-5 ml ampoules.

## Claims

1. Derivatives of 2-amino-4-phenyl-4-oxo-butyric acid which act as kynureninase enzyme inhibitors and/or kynurenine-3-hydroxylase enzyme inhibitors, having the following formula (I): 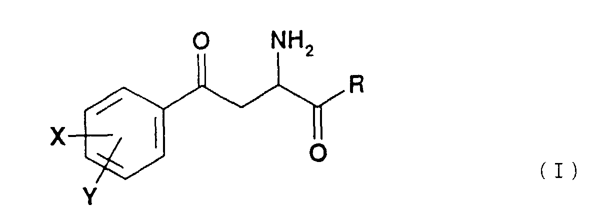 wherein
each of the groups X and Y is, independently, hydrogen, halogen, trifluoromethyl, hydroxy, C₁-C₆ alkyl, benzyl, C₆-C₁₀ aryl, -OR', -SR', 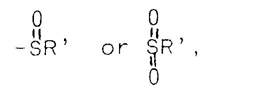 in which R' is C₁-C₆ alkyl or benzyl; and
R is hydroxy, amino, hydroxylamine, -OR', -NHR', 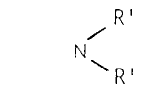 or - NHOR', in which R' is as defined above, either as single isomers or a mixture of isomers, and the pharmaceutically acceptable salts thereof, for use in the prevention and/or in the treatment of neurodegenerative diseases.

2. Use of compounds of formula (I) and pharmaceutically acceptable salts thereof according to claim 1 in the preparation of a medicament useful in the prevention and/or in the treatment of neurodegenerative diseases.

3. The use of a compound of formula (I) according to claim 1 or 2, either as single isomer or mixture of isomers or of a pharmaceutically acceptable salt thereof, in which the neurodegenerative disease is Huntington's chorea, Alzheimer's disease, Parkinson's disease, dementia caused by acquired immunodeficiency syndrome (AIDS), infarctual dementia, cerebral ischemia, cerebral hypoxia or epilepsy.

4. A derivative according to claim 1, which is a compound selected from the group consisting of:
2-amino-4-phenyl-4-oxo-butyric acid;
2-amino-4-(2'-methoxyphenyl)-4-oxo-butyric acid;
2-amino-4-(2'-fluorophenyl)-4-oxo-butyric acid;
2-amino-4-(4'-methoxyphenyl)-4-oxo-butyric acid;
2-amino-4-(2'-methylphenyl)-4-oxo-butyric acid;
2-amino-4-(3'-methoxyphenyl)-4-oxo-butyric acid;
2-amino-4-(2'-trifluoromethylphenyl)-4-oxo-butyric acid;
2-amino-4-(3'-trifluoromethylphenyl)-4-oxo-butyric acid;
2-amino-4-(4'-trifluoromethylphenyl)-4-oxo-butyric acid;
2-amino-4-(4'-chlorophenyl)-4-oxo-butyric acid;
2-amino-4-(3'-chlorophenyl)-4-oxo-butyric acid;
2-amino-4-(2'-chlorophenyl)-4-oxo-butyric acid;
2-amino-4-(3'-fluorophenyl)-4-oxo-butyric acid;
2-amino-4-(2'-methoxy-5'-fluorophenyl)-4-oxo-butyric acid;
2-amino-4-(3',4'-dichlorophenyl)-4-oxo-butyric acid;
2-amino-4-(2'-methoxy-5'-chlorophenyl)-4-oxo-butyric acid; and
2-amino-4-(2'-methoxy-5'-bromophenyl)-4-oxo-butyric acid;
either as a single isomer or a mixture of isomers, and the pharmaceutically acceptable salts thereof, for use in the prevention and/or in the treatment of neurodegenerative diseases.

5. A compound of formula (IA) 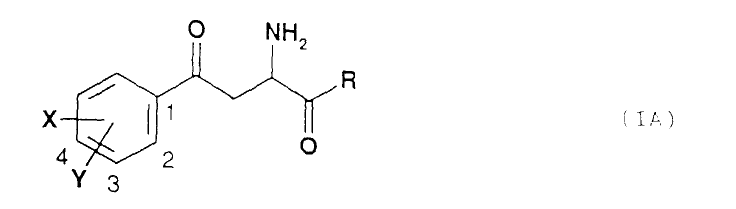 wherein
each of the groups X and Y is, independently, hydrogen, halogen, trifluoromethyl, hydroxy, C₁-C₆, alkyl, benzyl, C₆-C₁₀ aryl, -OR', -SR', 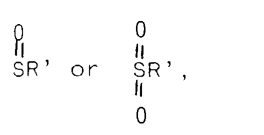 in which R' is C₁-C₆ alkyl or benzyl; and
R is hydroxy, -OR', amino, -NHR', 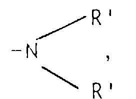 hydroxylamine or -NHOR', in which R' is as defined above; provided that R is not hydroxy when:
(i) X and Y are simultaneously hydrogen; or
(ii) X and Y are in positions 3 and 4 of the phenyl ring and are simultaneously a hydroxy group or a -OR' group in which R' is methyl; or
(iii) one of the groups X and Y is hydrogen and the other is in position 4 of the phenyl ring and is hydroxy, chlorine, fluorine, methyl, n-propyl, or methoxy;
and provided that R is not -OR', in which R' is C₁-C₆ alkyl, when X and Y are both hydrogen or methoxy;
either as single isomer or as a mixture of isomers and the pharmaceutically acceptable salts thereof.

6. A compound of formula (IA) according to claim 5, wherein
R is hydroxy and wherein
(a) one of the groups X and Y is hydrogen and the other is C₁-C₆ alkyl or trifluoromethyl in position 2, 3 or 4 of the phenyl ring, provided that when the C₁-C₆ alkyl is in position 4 of the phenyl ring, it is neither methyl nor n-propyl; or
(b) one of the groups X and Y is hydrogen and the other is a halogen atom in position 2, 3 or 4 of the phenyl ring, provided that when the halogen is in position 4 of the phenyl ring, it is neither chlorine nor fluorine; or
(c) one of the groups X and Y is hydrogen and the other is -OR', in which R' is C₁-C₆ alkyl, in position 2, 3 or 4 of the phenyl ring, provided that when -OR' is in position 4 of the phenyl ring, the C₁-C₆ alkyl is not methyl; or
(d) one of the groups X and Y is OR' in which R' is C₁-C₆ alkyl and the other is halogen;
either as single isomer or as mixture of isomers and the pharmaceutically acceptable salts thereof.

7. A compound according to claim 5 in form of a single isomer or of a mixture of isomers, which is a compound selected from the group consisting of:
2-amino-4-(2'-methoxyphenyl)-4-oxo-butyric acid,
2-amino-4-(3'-methoxyphenyl)-4-oxo-butyric Acid;
2-amino-4-(2'-fluorophenyl)-4-oxo-butyric acid;
2-amino-4-(3'-fluorophenyl)-4-oxo-butyric acid;
2-amino-4-(2'-chlorophenyl)-4-oxo-butyric acid;
2-amino-4-(3'-chlorophenyl)-4-oxo-butyric acid;
2-amino-4-(3',4'-dichlorophenyl)-4-oxo-butyric acid;
2-amino-4-(2'-methylphenyl)-4-oxo-butyric acid;
2-amino-4-(2'-trifluoromethylphenyl)-4-oxo-butyric acid;
2-amino-4-(3'-trifluoromethylphenyl)-4-oxo-butyric acid;
2-amino-4-(4'-trifluoromethylphenyl)-4-oxo-butyric acid;
2-amino-4-(2'-methoxy-5'-bromophenyl)-4-oxo-butyric acid;
2-amino-(2'-methoxy-5'-chlorophenyl)-4-oxo-butyric acid;
2-amino-4-(2'-methoxy-5'-fluorophenyl)-4-oxo-butyric acid;
and the pharmaceutically acceptable salts thereof.

8. A process for preparing a compound of formula (IA) according to claim 5 which comprises:
(A) reaction of a compound of formula (II) 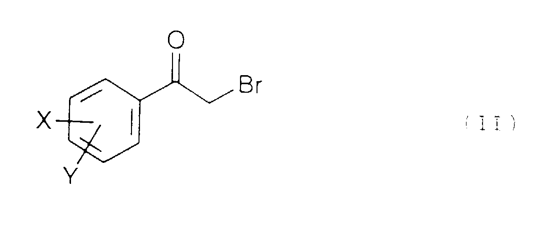 wherein X and Y are as defined in formula (IA) according to claim 5, with an alkali metal or alkaline-earth metal salt of a compound of formula (III) 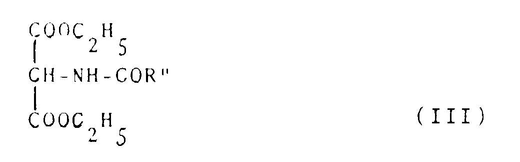 wherein R" is hydrogen or methyl, so obtaining a compound of formula (IV) 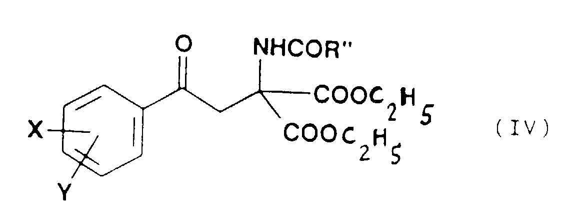 wherein X, and R" are as defined above;
(B) treatment of a compound of formula (IV) with concentrated halogenidric acid, so obtaining a compound of formula (IA) wherein X and Y are as defined above and R is hydroxy;
(C) optional conversion of a compound of formula (IA) into another compound of formula (IA) in which R is other than hydroxy;
(D) optional salification of a compound of formula (IA);
(E) optional separation of an isomeric mixture of a compound of formula (IA) into single isomers.

9. A process for preparing a single (R) or (S) enantiomer of a compound of formula (IA) according to claim 5, which comprises:
a) reacting a compound of formula (V) 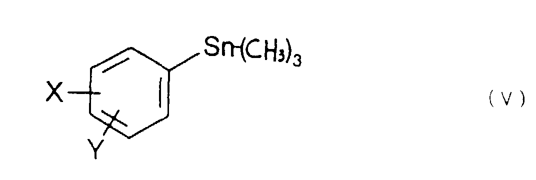 wherein
X and Y are as defined in formula (IA) according to claim 5, with a single (R) or (S) enantiomer of a compound of formula (VI) 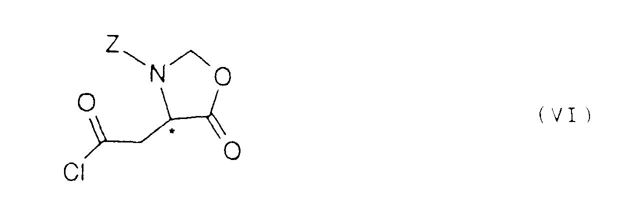 wherein
Z is a suitable amino protecting group, so obtaining a single (R) or (S) enantiomer of a compound of formula (VII) 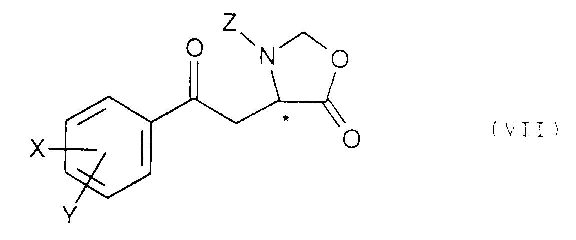 wherein
X, Y and Z are as defined above;
b) deprotecting a compound of formula (VII) so obtaining a single (R) or (S) enantiomer of a compound of formula (VIII) 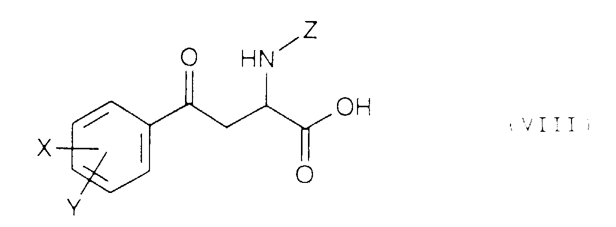 wherein
X, Y and Z are as defined above; and
c) further deprotecting a compound of formula (VIII) so obtaining a single (R) or (S) enantiomer of a compound of formula (lA) which, depending on the reaction conditions, is a free amino acid or its salt; the (R) or (S) configuration of a compound of formula (VI) being retained throughout the whole process leading to the compounds of formula (lA).

10. A process for preparing a compound of formula (IA) according to claim 5, as single (R) or (S) enantiomer or as a racemic mixture, which comprises:
a') reacting a compound of formula (IX) 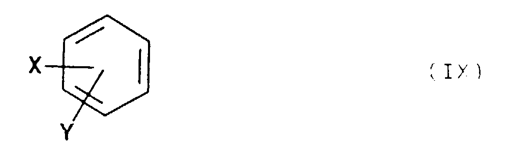 wherein
X and Y are as defined in formula (lA) according to claim 5, with a compound of formula (X) 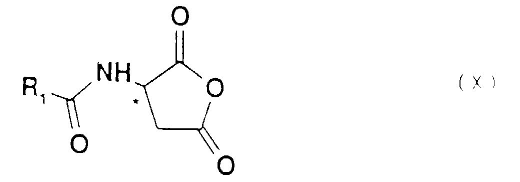 wherein
R, is hydrogen, methyl, trifluoromethyl, C₁-C₆ alkoxy or benzyloxy, either as a single (R) or (S) enantiomer or as racemic mixture, so obtaining a compound of formula (XI) 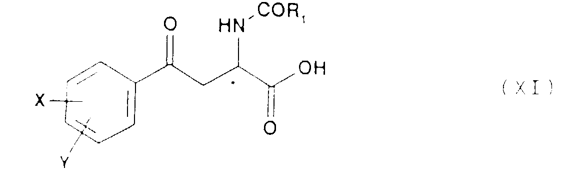 wherein
X, Y and R₁ are as defined above, either as a single (R) or (S) enantiomer or as racemic mixture;
b') converting a compound of formula (XI) either as a single (R) or (S) enantiomer or as racemic mixture into a single (R) or (S) enantiomer or racemic mixture of a compound of formula (IA) wherein X and Y are as defined above and R is hydroxy, and, if desired, converting a compound of formula (IA) wherein R is hydroxy into compound of formula (IA) wherein R is other than hydroxy.

11. A pharmaceutical composition comprising a carrier and/or a pharmaceutically acceptable diluent and, as an active substance, a compound of formula (I) according to claim 1, or of formula (IA) according to claim 5, either as a single isomer or as a mixture of isomers or a pharmaceutically acceptable salt thereof.

## Patentansprüche

1. Derivate der 2-Amino-4-phenyl-4-oxo-buttersäure, die als Kynureninase-Enzyminhibitoren und/oder Kynurenin-3-hydroxylase-Enzyminhibitoren fungieren und die folgende Formel (I) aufweisen: 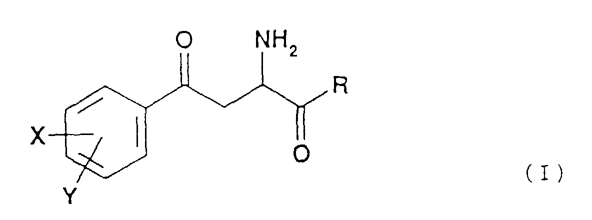 worin jede der Gruppen X und Y unabhängig voneinander Wasserstoff, Halogen, Trifluormethyl, Hydroxy, C₁-C₆-Alkyl, Benzyl, C₆-C₁₀-Aryl, -OR', -SR', 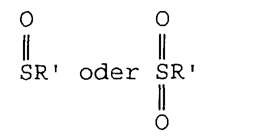 ist, worin R' C₁-C₆-Alkyl oder Benzyl ist; und
R ist Wasserstoff, Amino, Hydroxylamin, -OR', -NHR', 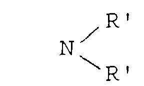 oder -NHOR', worin R' wie oben definiert ist, entweder als einzelne Isomere oder als Isomerenmischung, und pharmazeutisch annehmbare Salze davon, zur Verwendung zur Vorbeugung und/oder Behandlung von neurodegenerativen Erkrankungen.

2. Verwendung von Verbindungen der Formel (I) und pharmazeutisch annehmbaren Salzen davon gemäß Anspruch 1 zur Herstellung eines Medikaments, das zur Vorbeugung und/oder Behandlung von neurodegenerativen Erkrankungen geeignet ist.

3. Verwendung einer Verbindung der Formel (I) gemäß Anspruch 1 oder 2, entweder als Einzelisomer oder als Isomerenmischung, oder eines pharmazeutisch annehmbaren Salzes davon, worin die neurodegenerative Erkrankung Huntington's Chorea, Alzheimer, Parkinsonismus, durch erworbenes Immundefizienzsyndrom (AIDS) hervorgerufene Demenz, Infarktdemenz, zerebrale Durchblutungsstörungen, zerebrale Hypoxie oder Epilepsie ist.

4. Derivat gemäß Anspruch 1, das eine Verbindung ist, die ausgewählt ist aus:
2-Amino-4-phenyl-4-oxo-buttersäure;
2-Amino-4-(2'-methoxyphenyl)-4-oxo-buttersäure;
2-Amino-4-(2'-fluorphenyl) -4-oxo-buttersäure;
2-Amino-4-(4'-methoxyphenyl)-4-oxo-buttersäure;
2-Amino-4-(2'-methylphenyl)-4-oxo-buttersäure;
2-Amino-4-(3'-methoxyphenyl)-4-oxo-buttersäure;
2-Amino-4-(2'-trifluormethylphenyl)-4-oxo-buttersäure;
2-Amino-4-(3'-trifluormethylphenyl)-4-oxo-buttersäure;
2-Amino-4-(4'-trifluormethylphenyl)-4-oxo-buttersäure;
2-Amino-4-(4'-chlorphenyl)-4-oxo-buttersäure;
2-Amino-4-(3'-chlorphenyl)-4-oxo-buttersäure;
2-Amino-4-(2'-chlorphenyl)-4-oxo-buttersäure;
2-Amino-4-(3'-fluorphenyl)-4-oxo-buttersäure;
2-Amino-4-(2'-methoxy-5'-fluorphenyl)-4-oxo-buttersäure;
2-Amino-4-(3',4'-dichlorphenyl)-4-oxo-buttersäure;
2-Amino-4-(2'-methoxy-5'-chlorphenyl)-4-oxo-buttersäure; und
2-Amino-4-(2'-methoxy-5'-bromphenyl)-4-oxo-buttersäure;
entweder als Einzelisomer oder als Isomerenmischung, und die pharmazeutisch annehmbaren Salze davon, zur Verwendung in der Vorbeugung und/oder Behandlung von neurodegenerativen Erkrankungen.

5. Verbindung der Formel (IA) 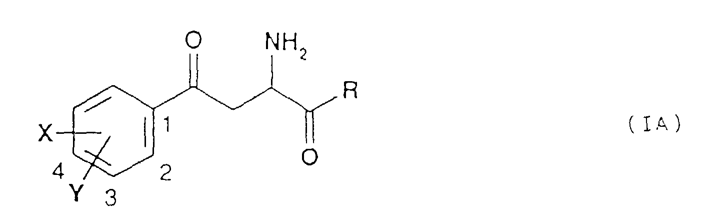 worin jede der Gruppen X und Y unabhängig voneinander Wasserstoff, Halogen, Trifluormethyl, Hydroxy, C₁-C₆-Alkyl, Benzyl, C₆-C₁₀-Aryl, -OR', -SR', 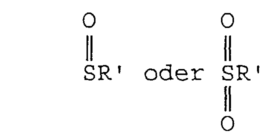 ist, worin R' C₁-C₆-Alkyl oder Benzyl ist; und R ist Hydroxy, -OR', Amino, -NHR', 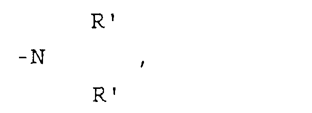 Hydroxylamin oder -NHOR', worin R' wie oben definiert ist, mit der Maßgabe, daß R nicht Hydroxy ist, wenn
(i) X und Y gleichzeigit Wasserstoff sind; oder
(ii) X und Y in den 3- und 4-Positionen des Phenylrings stehen und gleichzeitig Hydroxygruppen oder -OR'-Gruppen, worin R' Methyl ist, darstellen; oder
(iii) eine der Gruppen X und Y Wasserstoff ist, und die andere in der 4-Position des Phenylrings liegt und Hydroxy, Chlor, Fluor, Methyl, n-Propyl oder Methoxy ist;
und mit der Maßgabe, daß R nicht -OR' ist, worin R' C₁-C₆-Alkyl ist, wenn X und Y beide Wasserstoff oder Methoxy sind;
entweder als Einzelisomer oder als Isomerenmischung und die pharmazeutisch annehmbaren Salze davon.

6. Verbindung der Formel (IA) gemäß Anspruch 5, worin R Hydroxy ist und worin
(a) eine der Gruppen X und Y Wasserstofff und die andere C₁-C₆-Alkyl oder Trifluormethyl in der 2-, 3- oder 4-Position des Phenylrings ist, mit der Maßgabe, daß wenn die C₁-C₆-Alkylgruppe in der 4-Position des Phenylrings befindlich ist, diese weder Methyl oder Propyl ist; oder
(b) eine der Gruppen X und Y Wasserstoff ist, und die andere ist ein Halogenatom in der 2-, 3-, oder 4-Position des Phenylrings, mit der Maßgabe, daß wenn das Halogen in der 4-Position des Phenylrings befindlich ist, dieses weder Chlor noch Fluour ist; oder
(c) eine der Gruppen X und Y Wasserstoff ist, und die andere ist -OR', worin R' C₁-C₆-Alkyl darstellt, in der 2-, 3- oder 4-Position des Phenylrings, mit der Maßgabe, daß wenn -OR' in der 4-Position des Phenylrings befindlich ist, die C₁-C₆-Alkylgruppe nicht Methyl ist; oder
(d) eine der Gruppen X und Y OR' ist, worin R' C₁-C₆-Alkyl ist, und die andere ist ein Halogen;
entweder als Einzelisomer oder als Isomerenmischung, und die pharmazeutisch annehmbaren Salze davon.

7. Verbindung gemäß Anspruch 5 in Form eines Einzelisomers oder einer Isomerenmischung, die eine Verbindung ist, die ausgewählt ist aus:
2-Amino-4-(2'-methoxyphenyl)-4-oxo-buttersäure;
2-Amino-4-(3'-methoxyphenyl)-4-oxo-buttersäure;
2-Amino-4-(2'-fluorphenyl)-4-oxo-buttersäure;
2-Amino-4-(3'-fluorphenyl)-4-oxo-buttersäure;
2-Amino-4-(2'-chlorphenyl)-4-oxo-buttersäure;
2-Amino-4-(3'-chlorphenyl)-4-oxo-buttersäure;
2-Amino-4-(3'-4'-dichlorphenyl)-4-oxo-buttersäure;
2-Amino-4-(2'-methylphenyl)-4-oxo-buttersäure;
2-Amino-4-(2'-trifluormethylphenyl)-4-oxo-buttersäure;
2-Amino-4-(3'-trifluormethylphenyl)-4-oxo-buttersäure;
2-Amino-4-(4'-trifluormethylphenyl)-4-oxo-buttersäure;
2-Amino-4-(2'-methoxy-5'-bromphenyl)-4-oxo-buttersäure;
2-Amino-(2'-methoxy-5'-chlorphenyl)-4-oxo-buttersäure;
2-Amino-4-(2'-methoxy-5'-fluorphenyl)-4-oxo-buttersäure;
und die pharmazeutisch annehmbaren Salze davon.

8. Verfahren zur Herstellung einer Verbindung der Formel (IA) gemäß Anspruch 5, das folgendes umfaßt:
(A) Umsetzung einer Verbindung der Formel (II) 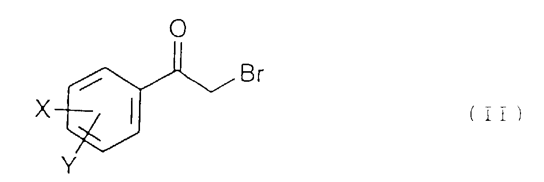 worin X und Y wie in Formel (IA) gemäß Anspruch 5 definiert sind, mit einem Alkalimetall- oder Erdalkalimetallsalz einer Verbindung der Formel (III) 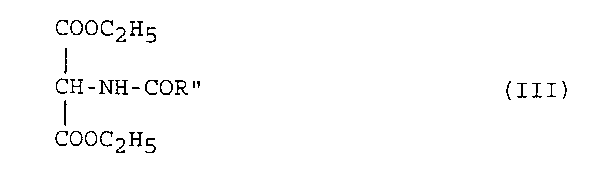 worin R" Wasserstoff oder Methyl ist, wodurch eine Verbindung der Formel (IV) erhalten wird, 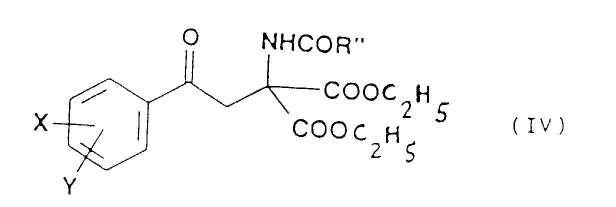 worin X, Y und R" wie oben definiert sind;
(B) Behandlung der Verbindung der Formel (IV) mit konzentrierter Halogensäure (halogenidric acid), wodurch eine Verbindung der Formel (IA) erhalten wird, worin X und Y wie oben definiert sind, und R ist Hydroxy;
(C) wahlweise Umwandlung der Verbindung der Formel (IA) in eine andere Verbindung der Formel (IA), worin R eine andere Gruppe ist als Hydroxy;
(D) wahlweise Versalzung der Verbindung der Formel (IA) ;
(E) wahlweise Trennung einer Isomerenmischung einer Verbindung der Formel (IA) in Einzelisomere.

9. Verfahren zur Herstellung eines einzelnen (R)- oder (S)-Enantiomers einer Verbindung der Formel (IA) gemäß Anspruch 5, das folgendes umfaßt:
a) Umsetzung einer Verbindung der Formel (V) 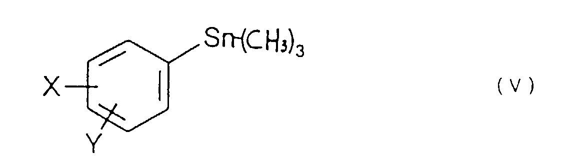 worin X und Y wie in Formel (IA) gemäß Anspruch 5 definiert sind, mit einem einzelnen (R)- oder (S)-Enantiomer einer Verbindung der Formel (VI) 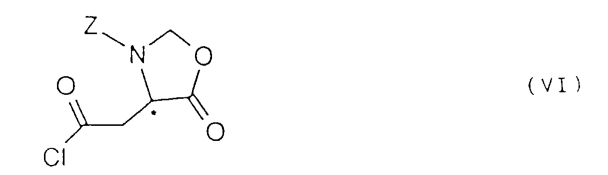 worin Z eine geeignete Aminoschutzgruppe ist, wodurch ein einzelnes (R)- oder (S)-Enantiomer einer Verbindung der Formel (VII) erhalten wird 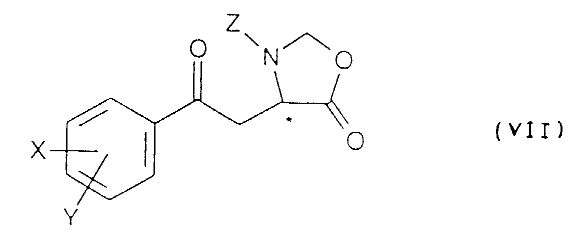 worin X, Y und Z wie oben definiert sind;
b) Entschützen der Verbindung der Formel (VII), wodurch ein einzelnes (R)- oder (S)-Enantiomer einer Verbindung der Formel (VIII) erhalten wird 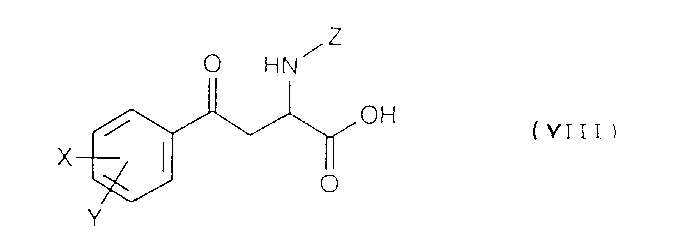 worin X, Y und Z wie oben definiert sind; und
c) weiteres Entschützen der Verbindung der Formel (VIII), wodurch ein einzelnes (R)- oder (S)- Enantiomer einer Verbindung der Formel (IA) erhalten wird, das in Abhängigkeit von den Reaktionsbedingungen eine freie Aminosäure oder deren Salz darstellt, die (R)- oder (S)- Konfiguration der Verbindung der Formel (VI) wird während des gesamten Verfahrens beibehalten und führt zu den Verbindungen der Formel (IA).

10. Verfahren zur Herstellung einer Verbindung der Formel (IA) gemäß Anspruch 5 als einzelnes (R)- oder (S)- Enantiomer oder als racemische Mischung, das folgendes umfaßt:
a') Umsetzung einer Verbindung der Formel (IX) 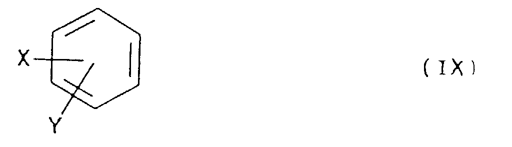 worin X und Y wie in Formel (IA) gemäß Anspruch 5 definiert sind, mit einer Verbindung der Formel (X) 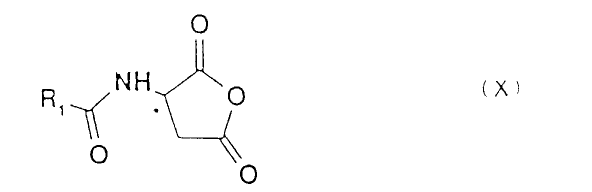 worin R Wasserstoff, Methyl, Trifluormethyl, C₁-C₆-Alkoxy oder Benzyloxy ist, entweder als einzelnes (R)- oder (S)- Enantiomer oder als racemische Mischung, wodurch eine Verbindung der Formel (XI) erhalten wird 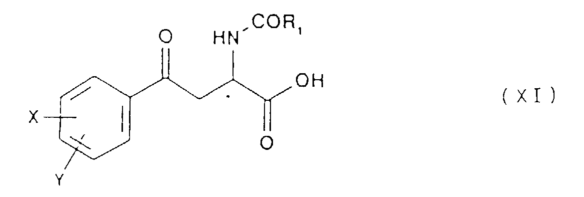 worin X, Y und R₁ wie oben definiert sind, entweder als einzelnes (R)- oder (S)- Enantiomer oder als racemische Mischung;
b') Umwandlung der Verbindung der Formel (XI) entweder als einzelnes (R)- oder (S)- Enantiomer oder als racemische Mischung in ein einzelnes (R)- oder (S)- Enantiomer oder eine racemische Mischung einer Verbindung der Formel (IA), worin X und Y wie oben definiert sind und R eine Hydroxygruppe darstellt, und bedarfsweise Umwandlung der Verbindung der Formel (IA), worin R Hydroxy ist, in eine Verbindung der Formel (IA), worin R eine andere Gruppe als eine Hydroxygruppe darstellt.

11. Pharmazeutische Zusammensetzung, die einen Träger und/oder ein pharmazeutisch annehmbares Verdünnungsmittel und als aktive Substanz eine Verbindung der Formel (I) gemäß Anspruch 1 oder der Formel (IA) gemäß Anspruch 5 entweder als Einzelisomer oder als Isomerenmischung oder ein pharmazeutisch annehmbares Salz davon umfaßt.

## Revendications

1. Dérivés de l'acide 2-amino-4-phényl-4-oxy-butyrique agissant comme inhibiteurs de l'enzyme kynureninase et/ou comme inhibiteurs de l'enzyme kynurenine-3-hydroxylase, ayant la formule (I) suivante: 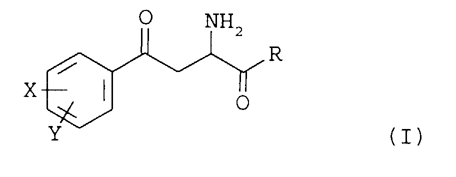 où
chaque groupe X et Y est, indépendamment, hydrogène, halogène, trifluorométhyl, hydroxy, C₁-C₆, alkyl, benzyl, C₆-C₁₀ aryl, -OR', -SR', -SOR', ou SO₂R', où R' est C₁-C₆ alkyl ou benzyl; et R est hydroxy, amino, hydroxylamine, -OR', -NHR', N(R')₂ ou NHOR', où R' est comme défini ci-avant, soit comme simples isomères, soit comme mélange d'isomères, et de leurs sels acceptables sur le plan pharmaceutique, pouvant être utilisés dans la prévention et/ou dans le traitement des maladies neuro-dégénératives.

2. Utilisation de composés de formule (I) et de leurs sels acceptables sur le plan pharmaceutique, selon la revendication 1, dans la préparation d'un médicament utile dans la prévention et/ou dans le traitement des maladies neuro-dégénératives.

3. L'utilisation d'un composé de formule (I) selon la revendication 1 ou 2, soit comme simple isomère, soit comme mélange d'isomères ou de leurs sels acceptables sur le plan pharmaceutique, où la maladie neuro-dégénérative est la chorée d'Huntington, la maladie d'Alzheimer, la maladie de Parkinson, la démence causée par le syndrome d'immunodéficience (SIDA), la démence d'infarctus, l'ischémie cérébrale, l'hypoxie cérébrale ou épilepsie.

4. Un dérivé selon la revendication 1, qui est un composé choisi dans le groupe suivant:
acide 2-amino-4-phényl-4-oxybutyrique;
acide 2-amino-4-(2'méthoxyphényl)-4-oxybutyrique;
acide 2-amino-4-(2'-fluorophényl)-4-oxybutyrique;
acide 2-amino-4-(4'-méthoxyphényl)-4-oxybutyrique;
acide 2-amino-4-(2'-méthylphényl)-4-oxybutyrique;
acide 2-amino-4-(3'-méthoxyphényl)-4-oxybutyrique;
acide 2-amino-4-(2'-trifluorométhylphényl)-4-oxybutyrique;
acide 2-amino-4-(3'-trifluorométhylphényl)-4-oxybutyrique;
acide 2-amino-4-(4'-trifluorométhylphényl)-4-oxybutyrique;
acide 2-amino-4-(4'-chlorophényl)-4-oxybutyrique;
acide 2-amino-4-(3'-chlorophényl)-4-oxybutyrique;
acide 2-amino-4-(2'-chlorophényl)-4-oxybutyrique;
acide 2-amino-4-(3'-fluorophényl)-4-oxybutyrique;
acide 2-amino-4-(2'-méthoxy-5'-fluorophényl)-4-oxybutyrique;
acide 2-amino-4-(3', 4'-dichlorophényl)-4-oxybutyrique;
acide 2-amino-4-(2'-méthoxy-5'-chlorophényl)-4-oxybutyrique;
acide 2-amino-4-(2'-méthoxy-5'-bromophényl)-4-oxybutyrique;
soit comme simple isomère soit comme mélange d'isomères, et leurs sels acceptables sur le plan pharmaceutique, pouvant être utilisés dans la prévention et/ou dans le traitement des maladies neuro-dégénératives.

5. Un composé de formule (IA) 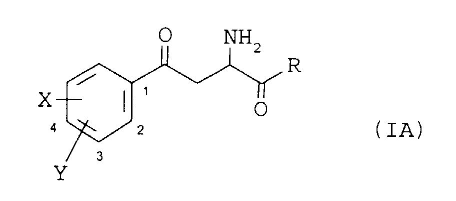 où
chaque groupe X et Y est, indépendamment, hydrogène, halogène, trifluorométhyl, hydroxy, C₁-C₆, alkyl, benzyl, C₆-C₁₀ aryl, -OR', -SR', SOR' ou SO₂R', où R' est C₁-C₆ alkyl ou benzyl; et
R est hydroxy, OR', amino, -NHR', -N(R')₂, hydroxylamine ou -NHOR', où R' est comme défini ci-avant; pourvu que R ne soit pas hydroxy lorsque:
(i) X et Y sont en même temps hydrogène; ou
(ii) X et Y sont en position 3 et 4 de l'anneau phényle et sont en même temps un groupe hydroxy ou un groupe -OR' dans lequel R' est méthyl; ou
(iii) un des groupes X et Y est hydrogène et l'autre est en position 4 de l'anneau phényle et est hydroxy, chlore, fluor, méthyl, n-propyl, ou méthoxy;
et pourvu que R ne soit pas -OR', dans lequel R' est C₁-C₆ alkyl, lorsque X et Y sont tous deux hydrogène ou méthoxy;
soit comme simple isomère soit comme mélange d'isomères et leurs sels acceptables sur le plan pharmaceutique.

6. Un composé de formule (IA) selon la revendication 5,
où
R est hydroxy et où
(a) un des groupes X et Y est hydrogène et l'autre est C₁-C₆ alkyl ou trifluorométhyl en position 2, 3 ou 4 de l'anneau phényle, pourvu que lorsque le C₁-C₆ alkyl est en position 4 de l'anneau phényle, il ne soit ni méthyl ni n-propyl; ou
(b) un des groupes X et Y est hydrogène et l'autre est un atome d'halogène en position 2, 3 ou 4 de l'anneau phényle, pourvu que lorsque l'halogène est en position 4 de l'anneau phényle, il ne soit ni chlore ni fluor; ou
(c) un des groupes X et Y est hydrogène et l'autre est -OR', dans lequel R' est C₁-C₆ alkyl, en position 2, 3 ou 4 de l'anneau phényle, pourvu que lorsque -OR' est en position 4 de l'anneau phényle le C₁-C₆ alkyl ne soit pas méthyl; ou
(d) un des groupes X et Y est OR', dans lequel R' est C₁-C₆ alkyl et l'autre est halogène;
soit comme simple isomère soit comme mélange d'isomères et leurs sels acceptables sur le plan pharmaceutique.

7. Un composé selon la revendication 5, sous forme de simple isomère ou d'un mélange d'isomères, qui est un composé choisi dans le groupe suivant:
acide 2-amino-4-(2'-méthoxyphényl)-4-oxybutyrique;
acide 2-amino-4-(3'-méthoxyphényl)-4-oxybutyrique;
acide 2-amino-4-(2'-fluorophényl)-4-oxybutyrique;
acide 2-amino-4-(3'-fluorophényl)-4-oxybutyrique;
acide 2-amino-4-(2'-chlorophényl)-4-oxybutyrique;
acide 2-amino-4-(3'-chlorolphényl)-4-oxybutyrique;
acide 2-amino-4-(3', 4'-dichlorophényl)-4-oxybutyrique;
acide 2-amino-4-(2'-méthylphényl)-4-oxybutyrique;
acide 2-amino-4-(2'-trifluorométhylphényl)-4-oxybutyrique;
acide 2-amino-4-(3'-trifluorométhylphényl)-4-oxybutyrique;
acide 2-amino-4-(4'-trifluorométhylphényl)-4-oxybutyrique;
acide 2-amino-4-(2'-méthoxy-5'-bromophényl)-4-oxybutyrique;
acide 2-amino-(2'-méthoxy-5'-chlorophényl)-4-oxybutyrique;
acide 2-amino-4-(2'-méthoxy-5'-fluorophényl)-4-oxybutyrique;
et leurs sels acceptables sur le plan pharmaceutique.

8. Un procédé pour préparer un composé de formule (IA) selon la revendication 5, qui comprend:
(A) la réaction d'un composé de formule (II) 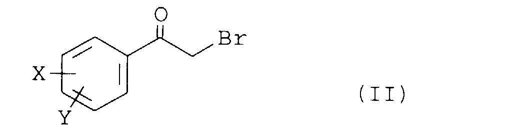 où X et Y sont comme définis dans la formule (IA) selon la revendication 5, avec un sel de métal alcalin ou de métal alcalino-terreux d'un composé de formule (III) 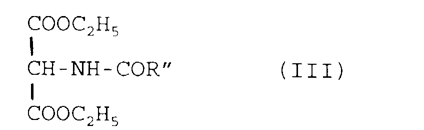 où R" est hydrogène ou méthyl, obtenant ainsi un composé de formule (IV) 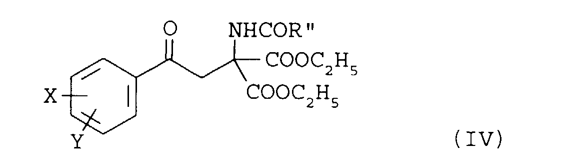 où X, Y et R" sont comme définis ci-avant;
(B) le traitement d'un composé de formule (IV) avec de l'acide halogénhydrique concentré, obtenant ainsi un composé de formule (IA), où X et Y sont comme définis ci-avant et R est hydroxy;
(C) la conversion facultative d'un composé de formule (IA) en un autre composé de formule (IA) où R est différent d'hydroxy;
(D) la salification facultative d'un composé de formule (IA) ;
(E) la séparation optionnelle d'un mélange isomérique d'un composé de formule (IA) en simple isomères.

9. Un procédé pour préparer un simple éniantomère (R) ou (S) d'un composé de formule (IA) selon la revendication 5, qui comprend:
a) la réaction d'un composé de formule (V), 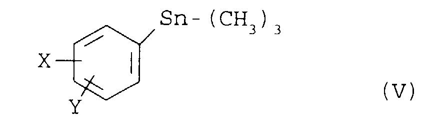 où
X et Y sont comme définis dans la formule (IA) selon la revendication 5, avec un simple éniantomère (R) ou (S) d'un composé de formule (VI) 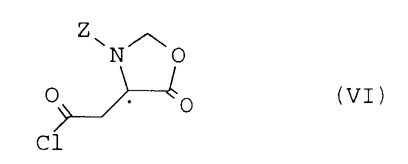 où
Z est un groupe protecteur aminique approprié, obtenant ainsi un simple éniantomère (R) ou (S) d'un composé de formule (VII) 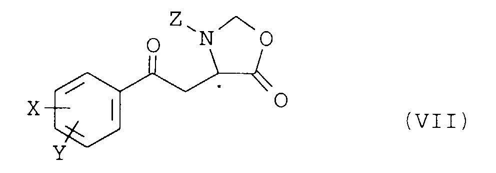 où
X, Y et Z sont comme définis ci-avant;
b) la déprotection d'un composé de formule (VII), obtenant ainsi un simple éniantomère (R) ou (S) d'un composé de formule (VIII) 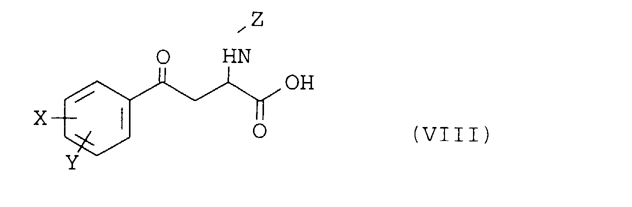 où
X, Y et Z sont comme définis ci-avant; et
c) la déprotection ultérieure d'un composé de formule (VIII), obtenant ainsi un simple éniantomère (R) ou (S) d'un composé de formule (IA) qui, selon les conditions de réaction, est un acide aminé libre ou son sel; la configuration (R) ou (S) d'un composé de formule (VI) étant maintenue tout au long du processus qui conduit aux composés de formule (IA).

10. Un procédé pour préparer un composé de formule (IA) selon la revendication 5, comme simple éniantomère (R) ou (S) ou comme mélange racémique, qui comprend:
a) la réaction d'un composé de formule (IX) 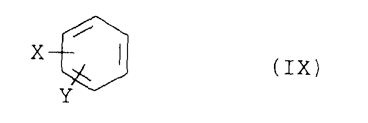 où
X et Y sont comme définis dans la formule (IA) selon la revendication 5, avec un composé de formule (X) 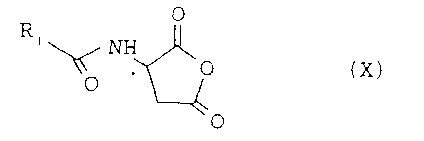 où
R est hydrogène, méthyl, trifluorométhyl, C₁-C₆ alcoxy ou benzyloxy, soit comme simple éniantomère (R) ou (S) soit comme mélange racémique, obtenant ainsi un composé de formule (XI) 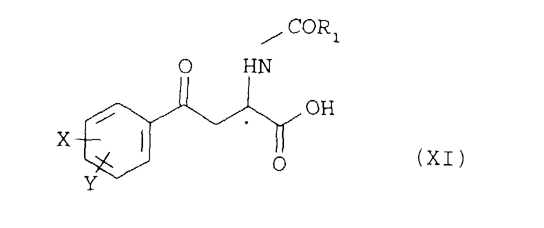 où
X, Y et R₁ sont comme définis ci-avant, soit comme simple éniantomère (R) ou (S) soit comme mélange racémique;
b) la conversion d'un composé de formule (XI), soit comme simple éniantomère (R) ou (S) soit comme mélange racémique, en un simple éniantomère (R) ou (S) ou en un mélange racémique d'un composé de formule (IA) où X et Y sont comme définis ci-avant, et R est hydroxy, et si nécessaire, en convertissant un composé de formule (IA) où R est hydroxy, en un composé de formule (IA) où R n'est pas hydroxy.

11. Une composition pharmaceutique qui comprend un carrier et/ou un diluant acceptable sur le plan pharmaceutique et, comme substance active, un composé de formule (I) selon la revendication 1, ou de formule (IA) selon la revendication 5, soit comme simple isomère soit comme mélange d'isomères ou un sel acceptable sur le plan pharmaceutique.
